(19) Europäisches Patentamt
European Patent Office
Office européen des brevets

(11) Numéro de publication: **0 421 829 B1**

(12) **FASCICULE DE BREVET EUROPEEN**

(45) Date de publication de fascicule du brevet: **09.11.94**

(51) Int. Cl.⁵: **C07H 17/07**, C07H 17/075, C07H 17/04, A61K 31/70, C07D 311/12, C07D 311/14, C07D 311/16, C07D 311/18, C07D 311/22

(21) Numéro de dépôt: **90402403.1**

(22) Date de dépôt: **31.08.90**

(54) **Nouveaux benzopyranone-beta-D-thioxylosides, leur procédé de préparation et leur utilisation en thérapeutique.**

(30) Priorité: **22.09.89 FR 8912452**
**16.03.90 FR 9003401**

(43) Date de publication de la demande:
**10.04.91 Bulletin 91/15**

(45) Mention de la délivrance du brevet:
**09.11.94 Bulletin 94/45**

(84) Etats contractants désignés:
**AT BE CH DE DK ES FR GB GR IT LI LU NL SE**

(56) Documents cités:
**EP-A- 0 051 023**
**EP-A- 0 130 833**
**EP-A- 0 133 103**
**EP-A- 0 221 293**
**EP-A- 0 290 321**
**FR-A- 2 100 884**

**BERICHTE DER DEUTSCHEN CHEMISCHEN GESELLSCHAFT, vol. 34, 1901, pages 1693-1698;St.V. KOSTANECKI et al.: "Synthesen in der Chromongruppe"**

(73) Titulaire: **FOURNIER INDUSTRIE ET SANTE**
**38, avenue Hoche**
**F-75008 Paris (FR)**

(72) Inventeur: **Samreth, Soth**
**2, rue de la Deuxième Escadre**
**F-21600 Longvic (FR)**
Inventeur: **Barberousse, Véronique**
**15, Boulevard Eugène Spuller**
**F-21000 Dijon (FR)**
Inventeur: **Renaut, Patrice, 3, ruelle de Plombière**
**Hauteville - Les Dijon**
**F-21121 Fontaine Les Dijon (FR)**
Inventeur: **Bellamy, François**
**Rue Basse Cédex 11,**
**Saulon la Rue**
**F-21910 Saulon la Chapelle (FR)**
Inventeur: **Millet, Jean**
**15, rue du Tremblois,**
**Corcelles les Citeaux**
**F-21910 Saulon la Rue (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

(74) Mandataire: **Clisci, Serge et al**
**S.A. FEDIT-LORIOT & AUTRES**
**38, avenue Hoche**
**F-75008 Paris (FR)**

## Description

La présente invention concerne en tant que produits industriels nouveaux, les composés benzopyranone-$\beta$-D-thioxylosidesde formule I ci-dessous. Elle concerne également leur procédé de préparation et leur utilisation en thérapeutique, en tant qu'agents antithrombotiques veineux.

On a déjà proposé dans EP-B-0 051 023 des dérivés de benzoyl-phényl-osides et d'$\alpha$-hydroxybenzyl-phényl-osides en tant qu'agents anti-ulcéreux, anti-agrégants plaquettaires, antithrombotiques et oxygénateurs cérébraux.

On connaît également de EP-A-0 133 103 des benzyl-phényl-osides utiles en tant qu'agents hypocholestérolémiants et hypolipidémiants, certains de ces composés, en particulier le produit de l'exemple 1, présentant en outre des effets antithrombotiques.

On connaît enfin de EP-A-0 290 321 des dérivés de benzoyl-phényl-thioxylosides, $\alpha$-hydroxybenzyl-phényl-thioxylosides et benzyl-phényl-thioxylosides proposés en tant qu'agents antithrombotiques.

On vient à présent de trouver que les composés benzopyranone-$\beta$-D-thioxylosides selon l'invention, structurellement différents des produits connus de l'art antérieur, sont utiles dans le traitement et la prévention des maladies liées aux troubles circulatoires, notamment en tant qu'agents antithrombotiques veineux.

Les nouveaux produits selon l'invention sont caractérisés en ce qu'ils sont choisis parmi l'ensemble constitué par les benzopyranone-$\beta$-D-thioxylosides de formule :

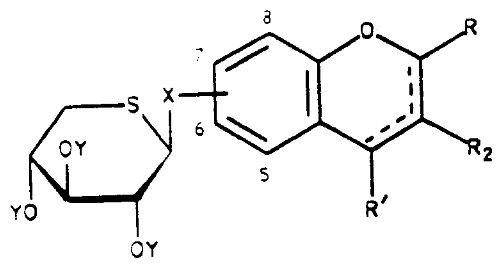

(I)

dans laquelle :

- l'un des substituants R ou R' représente un atome d'oxygène doublement lié au carbone cyclique et l'autre représente un groupe $R_1$,
- le symbole --- représente une double liaison conjuguée au groupe CO représenté par l'un des substituants R ou R',
- X représente un atome de soufre ou un atome d'oxygène,
- $R_1$ et $R_2$, identiques ou différents, représentent chacun un atome d'hydrogène, un groupe alkyle en $C_1$-$C_4$, un atome d'halogène, un groupe trifluorométhyle, un groupe phényle, $R_1$ et $R_2$, considérés ensemble, pouvant former avec le groupe benzopyranone auquel ils sont liés, un groupe 7,8,9,10-tétrahydrodibenzo[b,d]pyran-6-one ou un groupe 1,2,3,4-tétrahydro-9H-xanthène-9-one,
- les positions 5,6,7 et 8 représentent les possibilités de liaison de l'atome X au cycle benzopyranone ; et,
- Y représente l'atome d'hydrogène ou un groupe acyle aliphatique.

En d'autres termes, les nouveaux produits selon l'invention sont caractérisés en ce qu'ils sont choisis parmi l'ensemble constitué par les composés de formules :

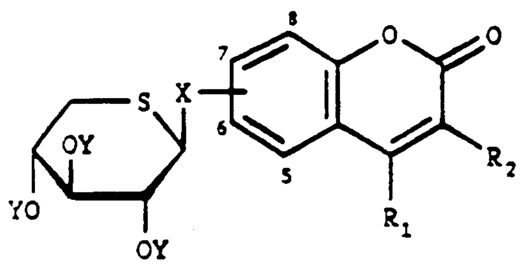

(I a)

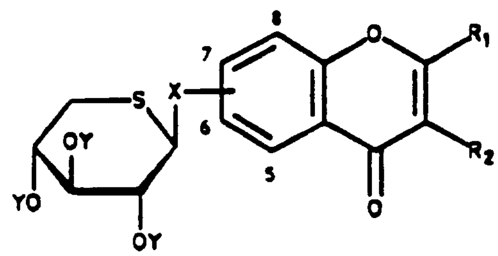

(I b)

dans lesquelles X, $R_1$, $R_2$ et Y ont les significations indiquées ci-dessus.

Les composés préférés selon l'invention sont les produits de formule I où X est lié en position 7 au cycle benzopyranone et où $R_1$ et $R_2$, identiques ou différents représentent chacun un atome d'hydrogène, un groupe alkyle en $C_1$-$C_4$, un atome d'halogène ou un groupe phényle.

Parmi les groupe acyles aliphatiques qui conviennent selon l'invention, on peut mentionner ceux qui renferment au total 2 à 5 atomes de carbone, le groupe acyle aliphatique préféré étant $CH_3CO$.

Par groupe alkyle en $C_1$-$C_4$, on entend ici un reste hydrocarboné, ramifié ou linéaire, contenant 1 à 4 atomes de carbone, le groupe alkyle préféré étant le groupe méthyle.

Par atome d' halogène, on entend ici les atomes de chlore, de fluor et de brome, l'atome d'halogène préféré étant l'atome de chlore.

Les composés de formule I et les composés acylés correspondants peuvent être préparés selon une réaction de glycosylation caractérisée en ce que :

(i) on fait réagir un composé de formule :

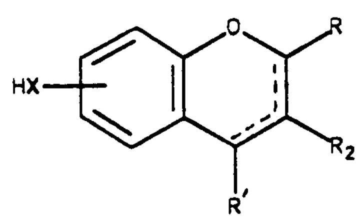

(II)

où X, R, R' et $R_2$ sont définis comme ci-dessus,

avec un dérivé du thioxylose choisi parmi l'ensemble comprenant :

(i) les halogénures d'acylthioxylosyle de formule :

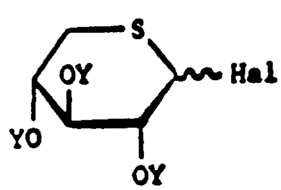

(III)

(ii) les thioxyloses peracylés de formule :

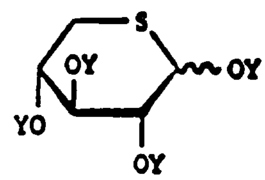

(IV)

et,

(iii) les trichloracétimidates d'acylthioxylosyle de formule :

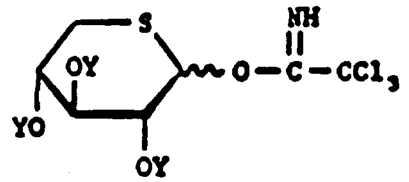

(V)

dans lesquelles Hal représente un atome d'halogène tel que Cl ou Br (l'atome de brome étant ici l'atome d'halogène préféré) et Y représente un groupe acyle, notamment un groupe acyle aliphatique comprenant un nombre total d'atomes de carbone de 2 à 5 et de préférence le groupe acétyle, dans un solvant inerte, à raison de 1 mole de II pour environ 0,6 à 1,2 moles de composé III, IV ou V, notamment en présence d'un accepteur d'acide et/ou d'un acide de Lewis, et,

(ii) si nécessaire, on soumet le composé de formule I ainsi obtenu où Y est un groupe acyle en $C_2$-$C_5$ à une réaction de désacylation à une température comprise entre 0 °C et la température de reflux du milieu réactionnel, dans un alcool inférieur en $C_1$-$C_4$ (de préférence le méthanol), en présence d'un alcoolate métallique (de préférence le méthylate de magnésium ou le méthylate de sodium), pour obtenir un composé de formule I où Y est H.

Les composés III, IV et V peuvent être de configuration $\alpha$ ou $\beta$ ou sous forme d'un mélange anomérique des deux configurations.

Les réactions de glycosylation des composés de formule II ont été conduites, soit au départ du composé III en présence d'un catalyseur comme les sels ou oxydes d'argent, de mercure ou de zinc, soit au départ du composé V en présence d'un acide de Lewis, notamment l'éthérate de trifluorure de bore ou le chlorure de zinc, soit encore au départ du composé IV en présence d'un acide de Lewis.

Selon un mode préféré de mise en oeuvre de l'invention, on préconise de condenser 1 mole du composé de formule II avec environ 1,1 à 1,2 moles d'halogénure d'acylthioxylosyle III dans un solvant inerte choisi parmi les solvants polaires ou apolaires (comme par exemple le diméthylformamide, le tétrahydrofuranne, le dioxanne, l'acétonitrile, le nitrométhane, le benzène, le toluène, les xylènes et leurs mélanges), en présence de cyanure mercurique.

On utilisera avantageusement le bromure de 2,3,4-tri-O-acétyl-5-thio-D-xylopyranosyle dans un mélange benzène/nitrométhane 1/1 (v/v) ou le dichloréthane, en présence de 1,1 à 1,3 moles de cyanure mercurique, à une température comprise entre 0 °C et la température de reflux du milieu réactionnel, de préférence à environ 40-50 °C, pendant 1 heure à 4 jours.

Selon un deuxième mode préféré de mise en oeuvre de l'invention, on préconise de condenser 1 mole du composé de formule II avec environ 1,1 à 1,2 moles d'halogénure d'acylthioxylosyle III dans un solvant inerte (comme par exemple le dichlorométhane ou l'acétonitrile) en présence d'imidazolate d'argent et de chlorure de zinc.

On utilisera avantageusement le bromure de 2,3,4-tri-O-acétyl-5-thio-D-xylopyranosyle dans du dichlorométhane ou un mélange dichlorométhane/acétonitrile en présence de 1,5 à 1,7 moles d'imidazolate d'argent et de 2 à 2,2 moles de chlorure de zinc, à une température comprise entre 0 °C et la température de reflux du milieu réactionnel, de préférence à environ 40-60 °C, pendant 24 à 48 heures.

Selon un troisième mode préféré de mise en oeuvre de l'invention, on préconise de condenser 1 mole du composé de formule II avec environ 0,6 à 1 mole d'halogénure d'acylthioxylosyle III dans un solvant

inerte (comme par exemple le toluène et/ou l'acétonitrile) en présence d'oxyde de zinc.

On utilisera avantageusement le bromure de 2,3,4-tri-O-acétyl-5-thio-D-xylopyranosyle dans un mélange toluène/acétonitrile, en présence de 0,5 à 1,2 moles d'oxyde de zinc, à une température comprise entre la température ambiante et la température de reflux du milieu réactionnel, de préférence à environ 40-60 °C, pendant 18 à 48 heures.

Selon un quatrième mode préféré de mise en oeuvre de l'invention, on préconise de condenser 1 mole du composé de formule II, avec environ 1,1 à 1,3 moles de trichloracétimidate d'acylthioxylosyle dans un solvant inerte (comme par exemple le dichlorométhane ou l'acétonitrile) en présence d'éthérate de trifluorure de bore ou de chlorure de zinc.

On utilisera avantageusement le trichloracétimidate de 2,3,4-tri-O-acétyl-5-thio-α-D-xylopyranosyle dans du dichlorométhane, en présence de 0,1 à 0,4 mole d'éthérate de trifluorure de bore en solution dans du dichlorométhane ou de l'acétonitrile, ou en présence de chlorure de zinc, à une température comprise entre -40 °C et la température ambiante (15-25 °C), de préférence à environ -20 °C à 0 °C, pendant 1 à 5 heures.

La réaction de glycosylation conduit dans tous les cas à un mélange des isomères de configuration α et β en proportions variables.

L'isomère de configuration β est isolé selon les méthodes connues de l'homme de l'art, comme par exemple la cristallisation fractionnée ou la chromatographie, notamment la "flash chromatography" [i.e. chromatographie sur colonne de silice et sous pression selon la technique décrite par W.C. STILL et al. dans J. Org. Chem. (1978), 42 (N° 14) 2923].

Les dérivés obtenus sont soumis, le cas échéant, à une désacylation, plus particulièrement à une désacétylation, qui est réalisée à une température comprise entre 0 °C et la température de reflux du milieu réactionnel dans un alcool inférieur en $C_1$-$C_4$, en présence de l'alcoolate métallique correspondant. De préférence, on choisira le méthanol comme alcool inférieur et le méthanolate de sodium ou de magnésium comme alcoolate métallique.

La réaction de désacylation peut éventuellement être conduite après glycosylation sans isoler le composé acylé intermédiaire formé.

On peut également réaliser la réaction de désacylation par voie enzymatique, par exemple par action de l'estérase de foie de porc.

Pour accéder aux composés intermédiaires de formule II où X = S on préconise :

(i) de condenser, en milieu basique fort, le chlorure de diméthyl-amino-thiocarbamoyle, de formule :

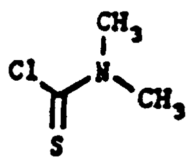

(VI)

sur un composé de formule :

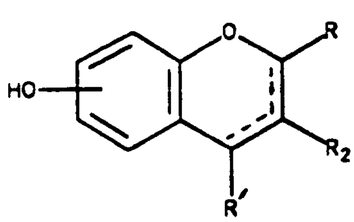

(IIa)

où R, R' et $R_2$ ont les significations indiquées ci-dessus, pour obtenir un composé de formule :

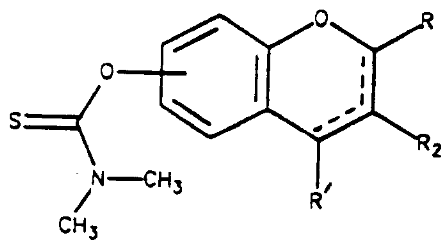

(VII)

où R, R' et $R_2$ ont les significations indiquées ci-dessus,

(ii) soumettre le composé de formule VII ainsi obtenu à un réarrangement de Newmann (J. Org. Chem. (1966) 31, p 3980), par chauffage, pour obtenir un composé de formule :

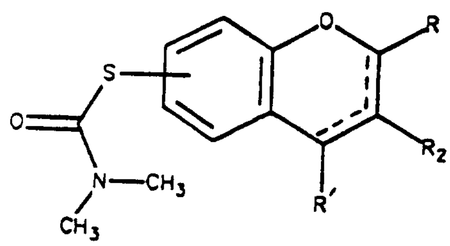

(VIII)

où R, R' et $R_2$ ont les significations indiquées ci-dessus,

(iii) traiter le composé de formule VIII ainsi obtenu par un alcoolate métallique, de préférence le méthanolate de sodium ou de magnésium, dans un alcool inférieur en $C_1$-$C_4$, de préférence le méthanol, le diméthylformamide ou le dioxanne pour obtenir un composé de formule II où X = S.

On peut également accéder aux composés intermédiaires de formule II ou X = S, R représente un atome d'oxygène doublement lié au carbone cyclique, R' représente un groupe $R_1$ et le symbole --- représente une double liaison conjuguée au groupe CO représenté par le substituant R, par substitution nucléophile d'un composé halogénobenzopyran-2-one approprié selon la méthode décrite par L. TESTAFERRI dans Tetrahedron Letters Vol. 21 p. 3099-3100 (1980).

Le composé intermédiaire 2-éthyl-7-hydroxy-4H-1-benzopyran-4-one est un composé nouveau et constitue un des objets de l'invention.

Les composés intermédiaires de formule II où X = S sont des composés nouveaux à l'exception du 7-mercapto-3-phényl-2H-1-benzopyran-2-one décrit dans GB-A-1154272.

Les composés de formule :

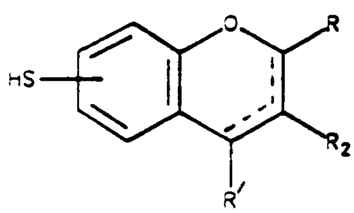

(IIb)

dans laquelle :

- l'un des substituants R ou R' représente un atome d'oxygène doublement lié au carbone cyclique et l'autre représente un groupe $R_1$,
- le symbole --- représente une double liaison conjuguée au groupe CO représenté par l'un des substituants R ou R',
- $R_1$ et $R_2$, identiques ou différents, représentent chacun un atome d'hydrogène, un groupe alkyle en $C_1$-$C_4$, un atome d'halogène, un groupe trifluorométhyle, un groupe phényle, à l'exception du groupe 3-phényle quand R représente un atome d'oxygène doublement lié au carbone cyclique, R' représente l'atome d'hydrogène, le groupe SH est lié en position 7 et le symbole --- représente une double

liaison conjuguée au groupe CO représenté par le substituant R, $R_1$ et $R_2$ considérés ensemble, pouvant former avec le groupe benzopyranone auquel ils sont liés un groupe 7,8,9,10-tétrahydrodibenzo[b,d]pyran-6-one ou un groupe 1,2,3,4-tétrahydro-9H-xanthène-9-one,

- les positions 5, 6, 7, et 8 représentent les possibilités de liaison de l'atome de soufre au cycle benzopyranone,

constituent donc un autre objet de l'invention.

Les composés intermédiaires de formule VIII sont des composés nouveaux.

Les composés de formule :

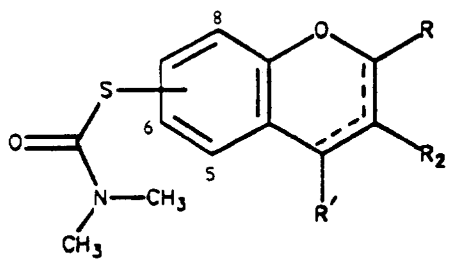

(VIII)

dans laquelle :

- $R_1$ et $R_2$, identiques ou différents, représentent chacun un atome d'hydrogène, un groupe alkyle en $C_1$-$C_4$, un atome d'halogène, un groupe trifluorométhyle, un groupe phényle, $R_1$ et $R_2$ considérés ensemble, pouvant former avec le groupe benzopyranone auquel ils sont liés un groupe 7,8,9,10-tétrahydrodibenzo[b,d]pyran-6-one ou un groupe 1,2,3,4-tétrahydro-9H-xanthène-9-one,
- les positions 5, 6, 7 et 8 représentent les possibilités de liaison de l'atome de soufre au cycle benzopyranone,

constituent donc un autre objet de l'invention.

Selon l'invention, on propose une composition thérapeutique caractérisée en ce qu'elle renferme, en association avec un excipient physiologiquement acceptable, au moins un composé choisi parmi l'ensemble constitué par les produits de formule I. Bien entendu, dans une telle composition l'ingrédient actif intervient en quantité thérapeutiquement efficace.

Les composés de formule I sont utiles en thérapeutique en tant qu'agents antithrombotiques. Ils sont notamment utiles dans la prévention et le traitement des troubles de la circulation veineuse.

Selon l'invention, on préconise l'utilisation d'une substance appartenant à l'ensemble des composés de formule I pour l'obtention d'un médicament antithrombotique destiné à une utilisation thérapeutique vis-à-vis des troubles de la circulation veineuse.

D'autres caractéristiques et avantages de l'invention seront mieux compris à la lecture qui va suivre, d'exemples de préparations, nullement limitatifs, donnés à titre d'illustration, et de résultats d'essais pharmacologiques.

Dans les exemples de préparation qui suivent, les composés ont été nommés en précisant la configuration $\alpha$ ou $\beta$ quand ladite configuration a été déterminée. Lorsque la configuration n'est pas indiquée, cela signifie que le produit correspondant est un mélange anomérique des configurations $\alpha$ et $\beta$ dans des proportions qui n'ont pas été déterminées.

## PREPARATION I

### Obtention du 4-éthyl-2-oxo-2H-1-benzopyran-7-yl 2,3,4-tri-O-acétyl-5-thio-$\beta$-D-xylopyranoside (exemple 1a)

Une suspension de 2,28 g ($12.10^{-3}$ mole) de 4-éthyl-7-hydroxy-2H-1-benzopyran-2-one, de 4,7 g ($13{,}2.10^{-3}$ mole) de bromure de 2,3,4-tri-O-acétyl-5-thio-D-xylopyranosyle et de tamis moléculaire 0,4 nm, dans 125 ml de toluène et 120 ml d'acétonitrile, est maintenue sous agitation, en présence de 3,28 g ($24.10^{-3}$ mole) de chlorure de zinc et de 4,2 g ($14.10^{-3}$ mole) d'imidazolate d'argent, à l'abri de la lumière, sous atmosphère inerte. Après chauffage à 55 °C pendant 24 h, on filtre le mélange réactionnel sur célite$^R$ - (i.e. silice diatomée pour filtration) dans l'acétate d'éthyle. Le filtrat est lavé au moyen d'une solution d'acide chlorhydrique 1N, d'une solution de soude 1N puis d'une solution saturée de chlorure de sodium, séché sur sulfate de magnésium et les solvants sont évaporés sous pression réduite. Après purification par chromatographie sur gel de silice en éluant au moyen d'un mélange acétate d'éthyle/toluène 1/6 (v/v) et précipitation

dans l'éther, on obtient 0,93 g rendement : 17 %) du produit attendu.
F = 189°C
$[\alpha]_D^{20}$ = - 73,8° (c = 0,25 ; $CHCl_3$)
En opérant de façon analogue on a préparé les produits suivants :

**4-méthyl-2-oxo-2H-1-benzopyran-6-yl 2,3,4-tri-O-acétyl-5-thio-β-D-xylopyranoside (exemple 2a)**

F = 179-184°C
$[\alpha]_D^{20}$ = - 47,9° (c = 0,33 ; $CHCl_3$)

**4-trifluorométhyl-2-oxo-2H-1-benzopyran-7-yl 2,3,4-tri-O-acétyl-5-thio-β-D-xylopyranoside (exemple 3a)**

F = 184°C
$[\alpha]_D^{20}$ = + 29,2° (c = 0,55 ; $CHCl_3$)

**4-méthyl-2-oxo-2H-1-benzopyran-8-yl 2,3,4-tri-O-acétyl-5-thio-β-D-xylopyranoside (exemple 4a)**

F = 220-223°C
$[\alpha]_D^{23}$ = - 121,9° (c = 0,21 ; $CHCl_3$)

**2-oxo-4-propyl-2H-1-benzopyran-7-yl 2,3,4-tri-O-acétyl-5-thio-β-D-xylopyranoside (exemple 5a)**

F = 165-167°C
$[\alpha]_D^{20}$ = - 71,2° (c = 0,11 ; $CHCl_3$)

**4-méthyl-2-oxo-2H-1-benzopyran-5-yl 2,3,4-tri-O-acétyl-5-thio-β-D-xylopyranoside (exemple 6a)**

F = 167°C
$[\alpha]_D^{22}$ = - 81° (c = 0,15 ; $CHCl_3$)

**4-méthyl-2-oxo-2H-1-benzopyran-7-yl 2,3,4-tri-O-acétyl-5-thio-β-D-xylopyranoside (exemple 16a)**

F = 193°C
$[\alpha]_D^{20}$ = - 72° (c = 0,5 ; $CHCl_3$)

**3-chloro-4-méthyl-2-oxo-2H-1-benzopyran-7-yl 2,3,4-tri-O-acétyl-5-thio-β-D-xylopyranoside (exemple 17a)**

F = 227°C
$[\alpha]_D^{20}$ = - 50,7° (c = 0,27 ; $CHCl_3$)

**4-méthyl-2-oxo-3-phényl-2H-1-benzopyran-7-yl 2,3,4-tri-O-acétyl-5-thio-β-D-xylopyranoside (exemple 18a)**

F = 210°C
$[\alpha]_D^{27,5}$ = - 56,5° (c = 0,1 ; $CHCl_3$)

**4-(1-méthyléthyl)-2-oxo-2H-1-benzopyran-7-yl 2,3,4-tri-O-acétyl-5-thio-β-D-xylopyranoside (exemple 19a)**

F = 144-145°C
$[\alpha]_D^{30}$ = - 26,4° (c = 0,1 ; $CH_3OH$)

**2-méthyl-4-oxo-4H-1-benzopyran-7-yl 2,3,4-tri-O-acétyl-5-thio-β-D-xylopyranoside (exemple 20a)**

F = 188°C
$[\alpha]_D^{23}$ = - 77,4° (c = 0,47 ; $CHCl_3$)

**2-éthyl-4-oxo-4H-1-benzopyran-7-yl 2,3,4-tri-O-acétyl-5-thio-β-D-xylopyranoside (exemple 21a)**

F = 150-151 °C
$[\alpha]_D^{21}$ = - 64 ° (c = 0,54 ; $CHCl_3$)

**2,3-diméthyl-4-oxo-4H-1-benzopyran-7-yl 2,3,4-tri-O-acétyl-5-thio-β-D-xylopyranoside (exemple 22a)**

F = 203-205 °C
$[\alpha]_D^{21}$ = - 65 ° (c = 0,6 ; $CHCl_3$)

**2-méthyl-4-oxo-4H-1-benzopyran-6-yl 2,3,4-tri-O-acétyl-5-thio-β-D-xylopyranoside (exemple 24a)**

F = 168-180 °C
$[\alpha]_D^{25}$ = - 81,9 ° (c = 0,3 ; $CHCl_3$)

**4-oxo-2-phényl-4H-1-benzopyran-7-yl 2,3,4-tri-O-acétyl-5-thio-β-D-xylopyranoside (exemple 25a)**

F = 215 °C
$[\alpha]_D^{24}$ = - 62 ° (c = 0,51 ; $CHCl_3$)

**3-bromo-2-méthyl-4-oxo-4H-1-benzopyran-7-yl 2,3,4-tri-O-acétyl-5-thio-β-D-xylopyranoside (exemple 28a)**

F = 192-194 °C
$[\alpha]_D^{21}$ = - 54 ° (c = 0,54 ; $CHCl_3$)

**PREPARATION II**

**Obtention du 4-méthyl-2-oxo-2H-1-benzopyran-5-yl 2,3,4-tri-O-acétyl-1,5-dithio-β-D-xylopyranoside - (exemple 7a)**

Une suspension de 420 mg ($2{,}2.10^{-3}$ mole) de 5-mercapto-4-méthyl-2H-1-benzopyran-2-one, de 970 mg ($2{,}7.10^{-3}$ mole) de bromure de 2,3,4-tri-O-acétyl-5-thio-D-xylopyranosyle, de 550 mg ($2{,}2.10^{-3}$ mole) de cyanure mercurique et de tamis moléculaire 0,4 nm dans 50 ml de nitrométhane et 50 ml de benzène est maintenue sous agitation à 45 °C sous atmosphère inerte pendant 24 h. Le mélange réactionnel est alors filtré sur Célite[R] dans l'acétate d'éthyle. Le filtrat est lavé au moyen d'une solution d'acide chlorhydrique 1N, d'une solution de soude 1N, puis d'une solution saturée de chlorure de sodium, séché sur sulfate de magnésium et évaporé sous pression réduite. On obtient, après purification par chromatographie sur gel de silice en éluant au moyen d'un mélange acétate d'éthyle/toluène 1/5 (v/v) puis précipitation dans l'éther 250 mg (rendement : 25 % ) du produit attendu.
F = 187 °C
$[\alpha]_D^{22}$ = + 34,5 ° (c = 0,11 ; $CHCl_3$)
En opérant de façon analogue on a préparé les produits suivants :

**4-méthyl-2-oxo-2H-1-benzopyran-8-yl 2,3,4-tri-O-acétyl-1,5-dithio-β-D-xylopyranoside (exemple 9a)**

F = 205 °C
$[\alpha]_D^{23}$ = + 86,25 ° (c = 0,3 ; $CHCl_3$)

**4-méthyl-2-oxo-2H-1-benzopyran-6-yl 2,3,4-tri-O-acétyl-1,5-dithio-β-D-xylopyranoside (exemple 10a)**

F = 139-140 °C
$[\alpha]_D^{23}$ = - 66,13 ° (c = 0,3 ; $CHCl_3$)

**7,8,9,10-tétrahydro-6-oxo-6H-dibenzo[b,d]pyran-3-yl 2,3,4-tri-O-acétyl-1,5-dithio-β-D-xylopyranoside (exemple 15a)**

F = 191 °C
$[\alpha]_D^{23}$ = + 14,5 ° (c = 0,3 ; $CHCl_3$)

**2-méthyl-4-oxo-4H-1-benzopyran-7-yl 2,3,4-tri-O-acétyl-1,5-dithio-β-D-xylopyranoside (exemple 23a)**

F = 171 °C
$[\alpha]_D^{21}$ = + 54,3 ° (c = 0,14 ; $CHCl_3$)

**2,3-diméthyl-4-oxo-4H-1-benzopyran-7-yl 2,3,4-tri-O-acétyl-1,5-dithio-β-D-xylopyranoside (exemple 26a)**

F = 169-173 °C
$[\alpha]_D^{30}$ = + 55,5 ° (c = 0,38 ; $CHCl_3$)

**2-éthyl-4-oxo-4H-1-benzopyran-7-yl 2,3,4-tri-O-acétyl-1,5-dithio-β-D-xylopyranoside (exemple 27a)**

F = 85-90 °C
$[\alpha]_D^{20}$ = + 58 ° (c = 0,5 ; $CHCl_3$)

**PREPARATION III**

**Obtention du 4-méthyl-2-oxo-2H-1-benzopyran-7-yl 2,3,4-tri-O-acétyl-1,5-dithio-β-D-xylopyranoside (exemple 8a)**

Un mélange de 8 g ($41,6.10^{-3}$ mole) de 7-mercapto-4-méthyl-2H-1-benzopyran-2-one, de 17,7 g ($50.10^{-3}$ mole) de bromure de 2,3,4-tri-O-acétyl-5-thio-α-D-xylopyranosyle et de 3,4 g ($42.10^{-3}$ mole) d'oxyde de zinc dans 180 ml de toluène et 180 ml d'acétonitrile est chauffé pendant 12 heures à 45 °C. Après filtration sur Célite[R] et lavage du résidu obtenu au moyen d'acétate d'éthyle, la phase organique est lavée au moyen d'une solution d'acide chlorhydrique 1N, puis d'une solution de soude 1N et enfin d'une solution saturée de chlorure de sodium. La phase organique obtenue est séchée sur sulfate de magnésium et évaporée sous pression réduite. On obtient, après précipitation par addition d'éther éthylique, 14,2 g (rendement : 73 %) d'une poudre jaune.
F = 168 °C
$[\alpha]_D^{22}$ = 46,4 ° (c = 0,7 ; $CHCl_3$)
En opérant de façon analogue on a préparé les produits suivants :

**4-trifluorométhyl-2-oxo-2H-1-benzopyran-7-yl 2,3,4-tri-O-acétyl-1,5-dithio-β-D-xylopyranoside (exemple 11a)**

F = 184 °C
$[\alpha]_D^{23}$ = + 80,25 ° (c = 0,5 ; $CHCl_3$)

**3-chloro-4-méthyl-2-oxo-2H-1-benzopyran-7-yl 2,3,4-tri-O-acétyl-1,5-dithio-β-D-xylopyranoside (exemple 12a)**

F = 160-162 °C
$[\alpha]_D^{23}$ = + 70,2 ° (c = 0,5 ; $CHCl_3$)

**4-éthyl-2-oxo-2H-1-benzopyran-7-yl 2,3,4-tri-O-acétyl-1,5-dithio-β-D-xylopyranoside (exemple 13a)**

F = 153 °C
$[\alpha]_D^{23}$ = + 28,11 ° (c = 0,5 ; $CHCl_3$)

**2-oxo-4-propyl-2H-1-benzopyran-7-yl 2,3,4-tri-O-acétyl-1,5-dithio-β-D-xylopyranoside (exemple 14a)**

F = 137 ° C
$[\alpha]_D^{23}$ = + 31,17 ° (c = 0,5 ; $CHCl_3$)

**PREPARATION IV**

**Obtention du 4-éthyl-2-oxo-2H-1-benzopyran-7-yl 5-thio-β-D-xylopyranoside (exemple 1)**

A une solution de 0,45 g (0,97.$10^{-3}$ mole) de 4-éthyl-2-oxo-2H-1-benzopyran-7-yl 2,3,4-tri-O-acétyl-5-thio-β-D-xylopyranoside dans 5 ml de méthanol, on ajoute 60 µl de méthylate de sodium (8 % de Na (p/v) dans le méthanol). Après 24 h sous agitation à température ambiante, le milieu réactionnel est neutralisé par addition de résine Amberlite$^R$ IR 120$H^+$, solubilisé avec du tétrahydrofuranne, filtré, traité au noir animal. Les solvants sont évaporés sous pression réduite puis après lyophilisation, on obtient 0,285 g (rendement : 87 % ) du produit attendu.
F = 192 ° C
$[\alpha]_D^{20}$ = - 69 ° (c = 0,21 ; diméthylsulfoxyde)
En opérant de façon analogue on a préparé les produits suivants :

**4-méthyl-2-oxo-2H-1-benzopyran-6-yl 5-thio-β-D-xylopyranoside (exemple 2)**

F = 109-113 ° C
$[\alpha]_D^{20}$ = - 63,3 ° (c = 0,24 ; diméthylsulfoxyde)

**4-trifluorométhyl-2-oxo-2H-1-benzopyran-7-yl 5-thio-β-D-xylopyranoside (exemple 3)**

F = 210-213 ° C
$[\alpha]_D^{20}$ = + 34,1 ° (c = 0,5 ; $CH_3OH$)

**4-méthyl-2-oxo-2H-1-benzopyran-8-yl 5-thio-β-D-xylopyranoside (exemple 4)**

F = 120-125 ° C
$[\alpha]_D^{20}$ = - 16 ° (c = 0,12 ; diméthylsulfoxyde)

**2-oxo-4-propyl-2H-1-benzopyran-7-yl 5-thio-β-D-xylopyranoside (exemple 5)**

F = 192 ° C
$[\alpha]_D^{24}$ = - 61,3 ° (c = 0,15 ; diméthylsulfoxyde)

**4-méthyl-2-oxo-2H-1-benzopyran-5-yl 5-thio-β-D-xylopyranoside (exemple 6)**

F = 184-188 ° C
$[\alpha]_D^{23}$ = - 85,2 ° (c = 0,11 ; diméthylsulfoxyde)

**4-méthyl-2-oxo-2H-1-benzopyran-5-yl 1,5-dithio-β-D-xylopyranoside (exemple 7)**

F = 203 ° C
$[\alpha]_D^{22}$ = + 28,3 ° (c = 0,12 ; $CH_3OH$)

**4-méthyl-2-oxo-2H-1-benzopyran-7-yl 1,5-dithio-β-D-xylopyranoside (exemple 8)**

F = 216 ° C
$[\alpha]_D^{23}$ = - 19,4 ° (c = 0,3 ; diméthylsulfoxyde)

**4-méthyl-2-oxo-2H-1-benzopyran-8-yl 1,5-dithio-β-D-xylopyranoside (exemple 9)**

F = 178 ° C
$[\alpha]_D^{23}$ = - 61,5 ° (c = 0,2 ; diméthylsulfoxyde)

**4-méthyl-2-oxo-2H-1-benzopyran-6-yl 1,5-dithio-β-D-xylopyranoside (exemple 10)**

F = 182 ° C
$[\alpha]_D^{20}$ = + 6,9 ° (c = 0,6 ; tétrahydrofuranne)

**4-trifluorométhyl-2-oxo-2H-1-benzopyran-7-yl 1,5-dithio-β-D-xylopyranoside (exemple 11)**

F = 178-180 ° C
$[\alpha]_D^{25}$ = + 40,8 ° (c = 0,26 ; $CH_3OH$)

**3-chloro-4-méthyl-2-oxo-2H-1-benzopyran-7-yl 1,5-dithio-β-D-xylopyranoside (exemple 12)**

F = 230 ° C
$[\alpha]_D^{22}$ = + 32,7 ° (c = 0,3 ; diméthylsulfoxyde)

**4-éthyl-2-oxo-2H-1-benzopyran-7-yl 1,5-dithio-β-D-xylopyranoside (exemple 13)**

F = 184 ° C
$[\alpha]_D^{25}$ = + 0,6 ° (c = 0,3 ; tétrahydrofuranne)

**2-oxo-4-propyl-2H-1-benzopyran-7-yl 1,5-dithio-β-D-xylopyranoside (exemple 14)**

F = 176-178 ° C
$[\alpha]_D^{25}$ = + 3,0 ° (c = 0,3 ; tétrahydrofuranne)

**7,8,9,10-tétrahydro-6-oxo-6H-dibenzo[b,d]pyran-3-yl 1,5-dithio-β-D-xylopyranoside (exemple 15)**

F = 182-183 ° C
$[\alpha]_D^{22}$ = + 20,6 ° (c = 0,3 ; diméthylsulfoxyde)

**4-méthyl-2-oxo-2H-1-benzopyran-7-yl 5-thio-β-D-xylopyranoside (exemple 16)**

F = 190-206 ° C
$[\alpha]_D^{20}$ = - 72 ° (c = 0,5 ; diméthylsulfoxyde)

**3-chloro-4-méthyl-2-oxo-2H-1-benzopyran-7-yl 5-thio-β-D-xylopyranoside (exemple 17)**

F = 208-210 ° C
$[\alpha]_D^{20}$ = - 22,9 ° (c = 0,24 ; diméthylsulfoxyde)

**4-méthyl-2-oxo-3-phényl-2H-1-benzopyran-7-yl 5-thio-β-D-xylopyranoside (exemple 18)**

F = 188-200 ° C
$[\alpha]_D^{22}$ = - 59,2 ° (c = 0,12 ; $CH_3OH$)

**4-(1-méthyléthyl)-2-oxo-2H-1-benzopyran-7-yl 5-thio-β-D-xylopyranoside (exemple 19)**

F = 186-190 ° C
$[\alpha]_D^{22}$ = - 74,3 ° (c = 0,14 ; $CH_3OH$)

**2-méthyl-4-oxo-4H-1-benzopyran-7-yl 5-thio-β-D-xylopyranoside (exemple 20)**

F = 193-195 ° C
$[\alpha]_D^{22}$ = - 92 ° (c = 0,5 ; méthanol)

**2-éthyl-4-oxo-4H-1-benzopyran-7-yl 5-thio-β-D-xylopyranoside (exemple 21)**

F = 130-137 °C
$[\alpha]_D^{21}$ = - 84 ° (c = 0,54 ; méthanol)

**2,3-diméthyl-4-oxo-4H-1-benzopyran-7-yl 5-thio-β-D-xylopyranoside (exemple 22)**

F = 177-194 °C
$[\alpha]_D^{21}$ = -88,6 ° (c = 0,45 ; tétrahydrofuranne)

**2-méthyl-4-oxo-4H-1-benzopyran-7-yl 1,5-dithio-β-D-xylopyranoside (exemple 23)**

F = 194-196 °C
$[\alpha]_D^{22}$ = + 19,1 ° (c = 0,2 ; diméthylsulfoxyde)

**2-méthyl-4-oxo-4H-1-benzopyran-6-yl 5-thio-β-D-xylopyranoside (exemple 24)**

F = 108 °C (décomposition : 200-240 °C)
$[\alpha]_D^{25}$ = - 107,7 ° (c = 0,3 ; méthanol)

**4-oxo-2-phényl-4H-1-benzopyran-7-yl 5-thio-β-D-xylopyranoside (exemple 25)**

F = 222 °C
$[\alpha]_D^{20}$ = - 90 ° (c = 0,5 ; tétrahydrofuranne)

**2,3-diméthyl-4-oxo-4H-1-benzopyran-7-yl 1,5-dithio-β-D-xylopyranoside (exemple 26)**

F = 204-208 °C
$[\alpha]_D^{30}$ = + 28,3 ° (c = 0,35 ; méthanol)

**2-éthyl-4-oxo-4H-1-benzopyran-7-yl 1,5-dithio-β-D-xylopyranoside (exemple 27)**

F = 155 °C
$[\alpha]_D^{20}$ = + 26,2 ° (c = 0,53 ; méthanol)

**3-bromo-2-méthyl-4-oxo-4H-1-benzopyran-7-yl 5-thio-β-D-xylopyranoside (exemple 28)**

F = 135-138 °C
$[\alpha]_D^{21}$ = - 43 ° (c = 0,5 ; diméthylsulfoxyde)

**PREPARATION V**

**Obtention du diméthylthiocarbamate de O-(4-méthyl-2-oxo-2H-1-benzopyran-5-yl)**

A une suspension de 1 g ($5{,}7.10^{-3}$ mole) de 5-hydroxy-4-méthyl-2H-1-benzopyran-2-one dans 10 ml d'eau et 10 ml d'acétone, on ajoute sous atmosphère inerte, 410 mg ($7{,}3.10^{-3}$ mole) de potasse. Après 10 minutes à température ambiante, on additionne à 0 °C, 770 mg $6{,}2.10^{-3}$ mole) de chlorure de diméthylthiocarbamoyle dans 10 ml d'acétone. On maintient sous agitation pendant 2 heures à température ambiante puis après évaporation de l'acétone, on précipite le dérivé attendu dans l'eau. On obtient 1,35 g (rendement : 90 %) du produit attendu.
F = 166-168 °C
En opérant de façon analogue on a préparé les produits regroupés dans les tableaux III et IV ci-après.

**PREPARATION VI**

**Obtention du diméthylthiocarbamate de S-(4-méthyl-2-oxo-2H-1-benzopyran-5-yl)**

Une solution de 3,7 g ($14.10^{-3}$ mole) de diméthylthiocarbamate de O-(4-méthyl-2-oxo-2H-1-benzopyran-5-yl) dans 50 ml de 1,2,3,4-tétrahydronaphthalène est maintenue à 220°C pendant 14 heures. Après refroidissement le produit attendu est précipité dans l'éther. Les cristaux obtenus sont rincés au cyclohexane et on obtient 2,95 g (rendement : 80 %) du produit attendu.
F = 129°C
En opérant de façon analogue on a préparé les produits regroupés dans les tableaux V et VI ci-après.

**PREPARATION VII**

**Obtention de l'ester méthylique de l'acide 3-(2-hydroxy-6-(diméthylamino carbonyl thio)phényl)-2-buténoïque**

A une solution de 2 g ($7{,}6.10^{-3}$ mole) de diméthylthiocarbamate de S-(4-méthyl-2-oxo-2H-1-benzopyran-5-yl) dans 20 ml de méthanol, on ajoute sous atmosphère inerte 4,4 ml de méthylate de sodium (8 % de Na (p/v) dans le méthanol). Après 4 heures à température ambiante le milieu réactionnel est hydrolysé sur un mélange glace/acide chlorhydrique et le précipité formé est filtré. On obtient 1,7 g (rendement : 76 %) du produit attendu.
F = 152°C

**PREPARATION VIII**

**Obtention du 7-mercapto-4-méthyl-2H-1-benzopyran-2-one**

A une solution de 1 g ($3{,}4.10^{-3}$ mole) de l'ester méthylique de l'acide 3-(2-hydroxy-6-(diméthylamino carbonyl thio)phényl)-2-buténoïque dans 10 ml de diméthylformamide anhydre, on ajoute, à 60°C, 4 ml de méthylate de sodium (8 % de Na (p/v) dans le méthanol). Après 6 heures à 60°C, le milieu réactionnel est hydrolysé sur un mélange acide chlorhydrique/glace. On obtient 0,550 g (rendement : 85 %) du produit attendu.
F = 136°C

**PREPARATION IX**

**Obtention du 7-mercapto-4-méthyl-2H-1-benzopyran-2-one**

Sous atmosphère d'azote, 26,3 g (0,1 mole) de diméthylthiocarbamate de S-(4-méthyl-2-oxo-2H-1-benzopyran-7-yl) sont mis en suspension dans 300 ml de méthanol . On ajoute, à température ambiante, 0,2 mole de méthylate de sodium (solution à 8 % de Na (p/v) dans le méthanol)et on chauffe à 45°C pendant 4 heures. On contrôle en chromatographie sur couche mince en éluant avec un mélange acétate d'éthyl/toluène 1/4 (v/v) la disparition du produit de départ. Après refroidissement, le milieu réactionnel est hydrolysé sur un mélange glace/acide chlorhydrique concentré et après 30 minutes d'agitation le précipité obtenu est filtré puis lavé à l'eau. Après séchage sur $P_2O_5$, on obtient 19,2 g (rendement ≈ 100 %) du produit attendu.
F = 132°C
En opérant de façon analogue on a préparé les produits regroupés dans les tableaux VII et VIII ci-après.

**PREPARATION X**

**Obtention de le 2-éthyl-7-(1-oxopropoxy)-3-(1-oxopropyl)-4H-1-benzopyran-4-one**

On maintient pendant 20 heures sous atmosphère inerte à une température de 170°C une solution de 5 g ($32{,}2.10^{-3}$ mole) de 1-(2,4-dihydroxyphényl) éthanone et de 4 g ($48{,}8.10^{-3}$ mole) d'acétate de sodium dans 40 ml d'anhydride de l'acide propanoïque. On hydrolyse le mélange réactionnel en présence de bicarbonate de sodium et le produit est extrait au moyen d'acétate d'éthyle puis lavé au moyen d'eau. La phase organique obtenue est séchée sur sulfate de magnésium. Le solvant est évaporé sous pression

réduite. Après addition de toluène on évapore à nouveau sous pression réduite les solvants restants. Après purification par chromatographie sur gel de silice en éluant au moyen d'un mélange hexane/acétate d'éthyle 6/1 (v/v) on obtient 2 g (rendement : 20 %) du produit attendu.
F = 84 °C

**PREPARATION XI**

**Obtention de le 2-éthyl-7-hydroxy-4H-1-benzopyran-4-one**

On maintient à une température de 150 °C pendant 9 heures une suspension de 6,5 g ($21,5.10^{-3}$ mole) de 2-éthyl-7-(1-oxopropoxy)-3-(1-oxopropyl)-4H-1-benzopyran-4-one et de 5 g ($47.10^{-3}$ mole) de carbonate de sodium dans 65 ml d'eau. On hydrolyse le mélange réactionnel au moyen d'une solution d'acide chlorhydrique 1N. Le produit est extrait au moyen d'acétate d'éthyle. Après purification par chromatographie sur gel de silice au moyen d'un mélange éther/dichlorométhane 1/2 (v/v) on obtient 2,45 g (rendement : 61 %) du produit attendu.
F = 189 °C

**PREPARATION XII**

**Obtention de la 7-acétyl-3-bromo-2-méthyl-4H-1-benzopyran-4-one**

A une solution de 8,8 g ($40.10^{-3}$ mole) de 7-acétyl-2-méthyl-4H-1-benzopyran-4-one dans 80 ml d'acide acétique on ajoute à 60 °C sous atmosphère inerte, 77,5 ml d'une solution de brome à 10 % dans l'acide acétique. Le mélange réactionnel est maintenu 2 heures à 60 °C puis est laissé 12 heures au repos. On concentre sous pression réduite puis on neutralise le résidu au moyen d'une solution saturée de bicarbonate de sodium. On extrait au moyen d'acétate d'éthyle. La phase organique est lavée à l'eau jusqu'à pH neutre, séchée sur sulfate de magnésium et concentrée à sec. Après chromatographie sur gel de silice en éluant au moyen d'un mélange dichlorométhane/méthanol 12/1 (v/v) on obtient 2,3 g (rendement : 19 %) du produit attendu.
F = 124 °C

**PREPARATION XIII**

**Obtention de le 3-bromo-7-hydroxy-2-méthyl-4H-1-benzopyran-4-one**

A une suspension de 2,3 g ($7,7.10^{-3}$ mole) de 7-acétyl-3-bromo-2-méthyl-4H-1-benzopyran-4-one dans 40 ml de méthanol, on ajoute 2,2 ml de méthanolate de sodium (8 % de Na (p/v) dans le méthanol) sous atmosphère inerte. Après 30 minutes le mélange réactionnel est hydrolysé par une solution glacée d'acide chlorhydrique. Le précipité formé est filtré puis lavé jusqu'à pH neutre. On obtient ainsi 1,9 g (rendement : 96 %) du produit attendu.
F = 305-310 °C (décomposition)
De façon non limitative, on a consigné un certain nombre de composés suivant l'invention dans les tableaux I et II ci-après.

L'activité anti-thrombotique des produits selon l'invention a été mise en évidence selon le protocole opératoire de thrombose veineuse suivant :
On réalise une stase veineuse sous hypercoagulation selon la technique décrite par WESSLER et al. (J. Applied Physiol. 1959, p. 943-946). L'agent hypercoagulant utilisé est, comme dans la technique décrite par J. HAUPMAN et al. (Thrombosis and Haemostasis 43 (2) 1980, P. 118), une solution de facteur X activé (Xa) fourni par la société dite Flow Laboratories (71 Knat pour 12,5 ml de sérum physiologique).

L'étude est réalisée sur des rats mâles Wistar, non à jeun, de 250 à 280 g répartis en lots de 10 animaux chacun. Les produits à tester sont administrés per os, en suspension dans le PEG 400. Une thrombose est induite 4 heures après ce traitement et le thrombus formé est prélevé et pesé.

Les résultats obtenus à la dose de 3 mg/kg p.o. ont été consignés dans les tableaux I et II.

**TABLEAU I**

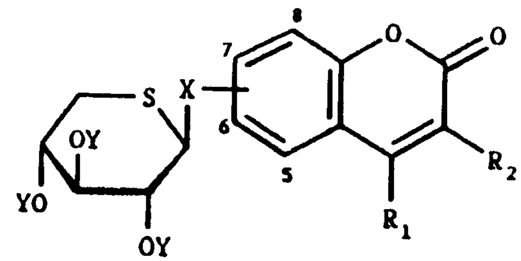

| Exemple | X | Position | $R_1$ | $R_2$ | Y | % d'inhibition à 3 mg/kg |
|---|---|---|---|---|---|---|
| 1a | O | 7 | $-CH_2-CH_3$ | -H | $-COCH_3$ | 65 |
| 1 | O | 7 | $-CH_2-CH_3$ | -H | -H | 87 |
| 2a | O | 6 | $-CH_3$ | -H | $-COCH_3$ | 53 |
| 2 | O | 6 | $-CH_3$ | -H | -H | 81 |
| 3a | O | 7 | $-CF_3$ | -H | $-COCH_3$ | 47 |
| 3 | O | 7 | $-CF_3$ | -H | -H | 80 |
| 4a | O | 8 | $-CH_3$ | -H | $-COCH_3$ | 51 |
| 4 | O | 8 | $-CH_3$ | -H | -H | 52 |
| 5a | O | 7 | $-(CH_2)_2-CH_3$ | -H | $-COCH_3$ | 46 |
| 5 | O | 7 | $-(CH_2)_2-CH_3$ | -H | -H | 26 |
| 6a | O | 5 | $-CH_3$ | -H | $-COCH_3$ | - |

**TABLEAU I (suite)**

| Exemple | X | Position | $R_1$ | $R_2$ | Y | % d'inhibition à 3 mg/kg |
|---|---|---|---|---|---|---|
| 6 | O | 5 | $-CH_3$ | -H | -H | 42 |
| 7a | S | 5 | $-CH_3$ | -H | $-COCH_3$ | - |
| 7 | S | 5 | $-CH_3$ | -H | -H | 38 |
| 8a | S | 7 | $-CH_3$ | -H | $-COCH_3$ | 65 |
| 8 | S | 7 | $-CH_3$ | -H | -H | 46 |
| 9a | S | 8 | $-CH_3$ | -H | $-COCH_3$ | - |
| 9 | S | 8 | $-CH_3$ | -H | -H | 31 |
| 10a | S | 6 | $-CH_3$ | -H | $-COCH_3$ | 32 |
| 10 | S | 6 | $-CH_3$ | -H | -H | 37 |
| 11a | S | 7 | $-CF_3$ | -H | $-COCH_3$ | - |
| 11 | S | 7 | $-CF_3$ | -H | -H | 36 |
| 12a | S | 7 | $-CH_3$ | -Cl | $-COCH_3$ | 32 |
| 12 | S | 7 | $-CH_3$ | -Cl | -H | 65 |
| 13a | S | 7 | $-CH_2-CH_3$ | -H | $-COCH_3$ | 58 |
| 13 | S | 7 | $-CH_2-CH_3$ | -H | -H | 54 |

## TABLEAU I (suite)

| Exemple | X | Position | $R_1$ | $R_2$ | Y | % d'inhibition à 3 mg/kg |
|---|---|---|---|---|---|---|
| 14a | S | 7 | $-(CH_2)_2-CH_3$ | -H | $-COCH_3$ | 25 |
| 14 | S | 7 | $-(CH_2)_2-CH_3$ | -H | -H | 43 |
| 15a | S | 7 | $-CH_2-CH_2-CH_2-CH_2-$ | | $-COCH_3$ | 23 |
| 15 | S | 7 | $-CH_2-CH_2-CH_2-CH_2-$ | | -H | 31 |
| 16a | O | 7 | $-CH_3$ | -H | $-COCH_3$ | 63 |
| 16 | O | 7 | $-CH_3$ | -H | -H | 63 |
| 17a | O | 7 | $-CH_3$ | -Cl | $-COCH_3$ | 67 |
| 17 | O | 7 | $-CH_3$ | -Cl | -H | 64 |
| 18a | O | 7 | $-CH_3$ | 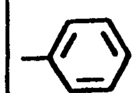 | $-COCH_3$ | 20 |
| 18 | O | 7 | $-CH_3$ | 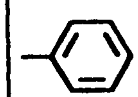 | -H | 43 |

**TABLEAU 1 (fin)**

| Exemple | X | Position | $R_1$ | $R_2$ | Y | % d'inhibition à 3 mg/kg |
|---|---|---|---|---|---|---|
| 19a | O | 7 | $-CH(CH_3)_2$ | -H | $-COCH_3$ | - |
| 19 | O | 7 | $-CH(CH_3)_2$ | -H | -H | 36 |
| A | produit de comparaison décrit à l'exemple 1 de EP-A-0133103 | | | | | 14 (1) |
| B | produit de comparaison décrit à l'exemple 97 de EP-B-0051023 | | | | | 5,5 (1) |
| C | produit de comparaison décrit à l'exemple 3 de EP-A-0290321 | | | | | 20 (2) |

Notes : (1) produit testé à 12,5 mg/kg p.o.
(2) produit testé à 3 mg/kg p.o.

## TABLEAU II

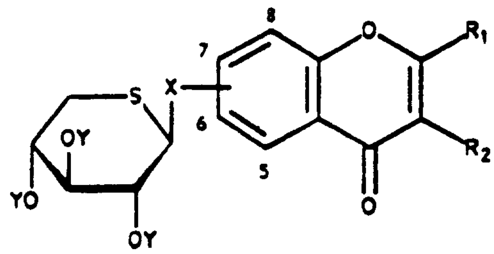

| n° exemple | X | Position | Y | R1 | R2 | % d'inhibition à 3 mg/kg |
|---|---|---|---|---|---|---|
| 20a | O | 7 | $-COCH_3$ | $-CH_3$ | -H | 20 |
| 20 | O | 7 | -H | $-CH_3$ | -H | 53 |
| 21a | O | 7 | $-COCH_3$ | $-C_2H_5$ | -H | 42 |
| 21 | O | 7 | -H | $-C_2H_5$ | -H | 70 |
| 22a | O | 7 | $-COCH_3$ | $-CH_3$ | $-CH_3$ | 66 |
| 22 | O | 7 | -H | $-CH_3$ | $-CH_3$ | 36 |
| 23a | S | 7 | $-COCH_3$ | $-CH_3$ | -H | - |
| 23 | S | 7 | -H | $-CH_3$ | -H | 26 |
| 24a | O | 6 | $-COCH_3$ | $-CH_3$ | -H | - |
| 24 | O | 6 | -H | $-CH_3$ | -H | 36 |
| 25a | O | 7 | $-COCH_3$ | $-C_6H_5$ | -H | - |
| 25 | O | 7 | -H | $-C_6H_5$ | -H | 28 |
| 26a | S | 7 | $-COCH_3$ | $-CH_3$ | $-CH_3$ | - |
| 26 | S | 7 | -H | $-CH_3$ | $-CH_3$ | 27 |
| 27a | S | 7 | $-COCH_3$ | $-C_2H_5$ | -H | 23 |
| 27 | S | 7 | -H | $-C_2H_5$ | -H | 26 |

TABLEAU II (fin)

| exemple | X | Position | Y | $R_1$ | $R_2$ | % d'inhibition |
|---|---|---|---|---|---|---|
| 28a | O | 7 | $-COCH_3$ | $-CH_3$ | -Br | 25 |
| 28 | O | 7 | - H | $-CH_3$ | -Br | 49 |
| A | Produit de comparaison décrit à l'exemple 1 de EP-A-0 133 103 | | | | | 14(1) |
| B | Produit de comparaison décrit à l'exemple 97 de EP-B-0 051023 | | | | | 5,5(1) |
| C | Produit de comparaison décrit à l'exemple 3 de EP-A-0 290 321 | | | | | 20(2) |

Notes : (1) produit testé à 12,5 mg/kg p.o.
(2) produit testé à 3 mg/kg p.o.

TABLEAU III

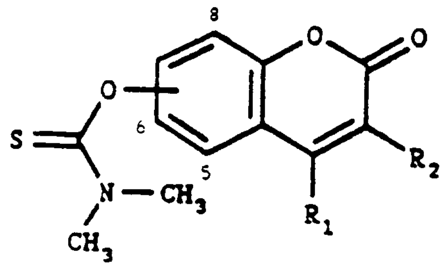

| Position | $R_1$ | $R_2$ | F (°C) |
|---|---|---|---|
| 5 | $-CH_3$ | -H | 166-168 |
| 7 | $-CH_3$ | -H | 216 |
| 8 | $-CH_3$ | -H | 194 |
| 6 | $-CH_3$ | -H | 164 |
| 7 | $-CF_3$ | -H | 160 |
| 7 | $-CH_3$ | -Cl | 184,5 |
| 7 | $-CH_2-CH_3$ | -H | 158-160 |
| 7 | $-CH_2-CH_2-CH_3$ | -H | 118-120 |
| 7 (1) | $-CH_2-CH_2-CH_2-CH_2-$ | | 159-160 |

Note : (1) diméthylthiocarbamate de O-(7,8,9,10-tétrahydro-6-oxo-6H-dibenzo[b,d]pyran-3-yl)

**TABLEAU IV**

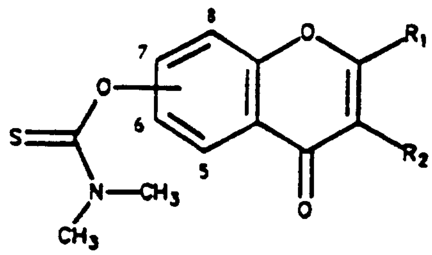

| Position | $R_1$ | $R_2$ | F (°C) |
|---|---|---|---|
| 7 | $-CH_3$ | -H | 137 |
| 7 | $-CH_3$ | $-CH_3$ | 160 |
| 7 | $-CH_2-CH_3$ | -H | 140 |

TABLEAU V

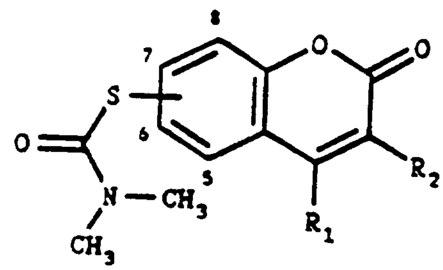

| Position | R1 | R2 | F (°C) |
|---|---|---|---|
| 5 | $-CH_3$ | -H | 129 |
| 7 | $-CH_3$ | -H | 154 |
| 8 | $-CH_3$ | -H | 154 |
| 6 | $-CH_3$ | -H | 137 |
| 7 | $-CF_3$ | -H | 138 |
| 7 | $-CH_3$ | -Cl | 229 |
| 7 | $-CH_2-CH_3$ | -H | 124 |
| 7 | $-CH_2-CH_2-CH_3$ | -H | 99-100 |
| 7 (1) | $-CH_2-CH_2-CH_2-CH_2-$ | | 132 |

Note : (1) diméthylthiocarbamate de S-(7,8,9,10-tétrahydro-6-oxo-6H-dibenzo[b,d]pyran-3-yl)

TABLEAU VI

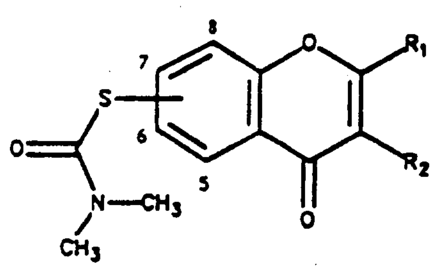

| Position | $R_1$ | $R_2$ | F (°C) |
|---|---|---|---|
| 7 | $-CH_3$ | -H | 164 |
| 7 | $-CH_3$ | $-CH_3$ | 138 |
| 7 | $-CH_2-CH_3$ | -H | 116 |

TABLEAU VII

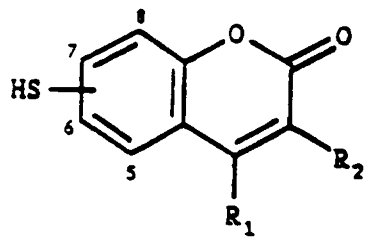

| Position | R1 | R2 | F (°C) |
|---|---|---|---|
| 7 | $-CH_3$ | -H | 136 |
| 7 | $-CH_3$ | -H | 132 |
| 8 | $-CH_3$ | -H | 114-115 |
| 6 | $-CH_3$ | -H | 138-140 |
| 7 | $-CF_3$ | -H | 115 |
| 7 | $-CH_3$ | -Cl | 153 |
| 7 | $-CH_2-CH_3$ | -H | 152 |
| 7 | $-CH_2-CH_2-CH_3$ | -H | 88-89 |
| 7 (1) | $-CH_2-CH_2-CH_2-CH_2-$ | | 139 |

Note : (1) 7,8,9,10-tétrahydro-3-mercapto-6H-dibenzo[b,d]pyran-6-one

TABLEAU VIII

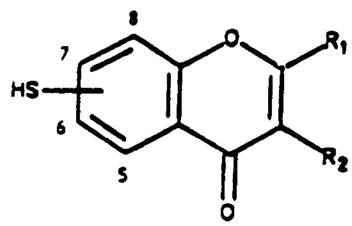

| POSITION | R1 | R2 | F (°C) |
|---|---|---|---|
| 7 | $-CH_3$ | -H | 120 |
| 7 | $-CH_3$ | $-CH_3$ | 122 |
| 7 | $-CH_2-CH_3$ | -H | 74 |

**Revendications**

**Revendications pour les Etats contractants suivants : AT, BE, CH, DE, DK, FR, GB, IT, LI, LU, NL, SE**

**1.** composé oside caractérisé en ce qu'il est choisi parmi l'ensemble comprenant les benzopyranone-$\beta$-D-thioxylosides de formule :

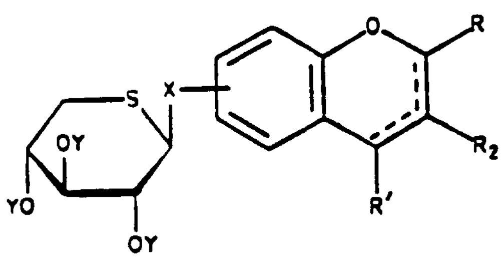

(I)

dans laquelle :

- l'un des substituants R ou R' représente un atome d'oxygène doublement lié au carbone cyclique et l'autre représente un groupe $R_1$,
- le symbole --- représente une double liaison conjugée au groupe CO représenté par l'un des substituants R ou R',
- X représente un atome de soufre ou un atome d'oxygène,
- $R_1$ et $R_2$, identiques ou différents, représentent chacun un atome d'hydrogène, un groupe alkyle en $C_1$-$C_4$, un atome d'halogène, un groupe trifluorométhyle, un groupe phényle, $R_1$ et $R_2$, considérés ensemble, pouvant former avec le groupe benzopyranone auquel ils sont liés, un groupe 7,8,9,10-tétrahydrodibenzo[b,d]pyran-6-one ou un groupe 1,2,3,4-tétrahydro-9H-xanthène-9-one ; et,

- Y représente l'atome d'hydrogène ou un groupe acyle aliphatique en $C_2$-$C_5$.

**2.** Composé oside selon la revendication 1 caractérisé en ce qu'il est choisi parmi l'ensemble comprenant les benzopyran-2-one-β-D-thioxylosides de formule :

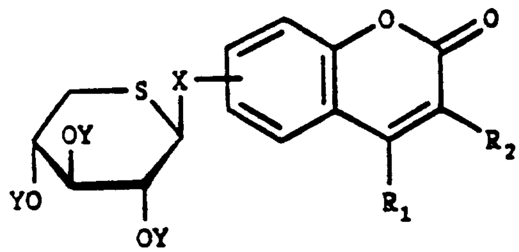

(I a)

dans laquelle :
- X représente un atome de soufre ou un atome d'oxygène,
- $R_1$ et $R_2$, identiques ou différents, représentent chacun un atome d'hydrogène, un groupe alkyle en $C_1$-$C_4$, un atome d'halogène, un groupe trifluorométhyle, un groupe phényle, $R_1$ et $R_2$, considérés ensemble, pouvant former avec le groupe benzopyran-2-one auquel ils sont liés, un groupe 7,8,9,10-tétrahydrodibenzo[b,d]pyran-6-one ; et,
- Y représente l'atome d'hydrogène ou un groupe acyle aliphatique.

**3.** Composé oside selon la revendication 1 caractérisé en ce qu'il est choisi parmi l'ensemble comprenant les benzopyran-4-one-β-D-thioxylosides de formule :

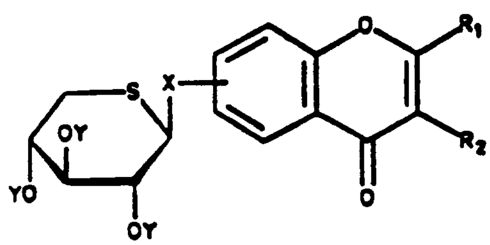

(Ib)

dans laquelle :
- X représente un atome de soufre ou un atome d'oxygène,
- $R_1$ et $R_2$, identiques ou différents, représentent chacun un atome d'hydrogène, un groupe alkyle en $C_1$-$C_4$, un atome d'halogène, un groupe trifluorométhyle, un groupe phényle, $R_1$ et $R_2$, considérés ensemble, pouvant former avec le groupe benzopyran-4-one auquel ils sont liés, un groupe 1,2,3,4-tétrahydro-9H-xanthène-9-one ; et,
- Y représente l'atome d'hydrogène ou un groupe acyle aliphatique en $C_2$-$C_5$.

**4.** Composé oside selon l'une quelconque des revendications 1 à 3, caractérisé en ce que X est lié en position 7 au cycle benzopyranone et $R_1$ et $R_2$, identiques ou différents, représentent chacun un atome d'hydrogène, un groupe alkyle en $C_1$-$C_4$, un atome d'halogène ou un groupe phényle.

**5.** Composé oside selon l'une quelconque des revendications 1 à 4, caractérisé en ce que Y représente le groupe $CH_3CO$.

**6.** 4-méthyl-2-oxo-2H-1-benzopyran-7-yl 1,5-dithio-β-D-xylopyranoside.

**7.** 3-chloro-4-méthyl-2-oxo-2H-1-benzopyran-7-yl 1,5-dithio-β-D-xylopyranoside.

**8.** 4-éthyl-2-oxo-2H-1-benzopyran-7-yl 5-thio-β-D-xylopyranoside.

9. Composition thérapeutique caractérisée en ce qu'elle renferme, en association avec un excipient physiologiquement acceptable, au moins un composé oside selon l'une quelconque des revendications 1 à 8.

10. Utilisation d'une substance selon l'une quelconque des revendications 1 à 8, pour l'obtention d'un médicament antithrombotique destiné à une utilisation thérapeutique vis-à-vis des troubles de la circulation veineuse.

11. Procédé de préparation d'un benzopyranone-$\beta$-D-thioxyloside de formule I selon la revendication 1 caractérisé en ce que :
(i) on fait réagir un composé de formule :

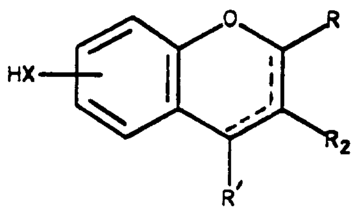

(II)

où X, R, R' et $R_2$ sont définis comme ci-dessus,
avec un dérivé du thioxylose choisi parmi l'ensemble comprenant :
(i)les halogénures d'acylthioxylosyle de formule :

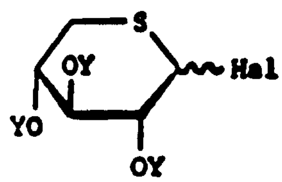

(III)

(ii) les thioxyloses peracylés de formule :

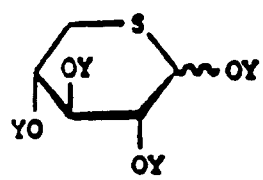

(IV)

et,
(iii) les trichloracétimidates d'acylthioxylosyle de formule :

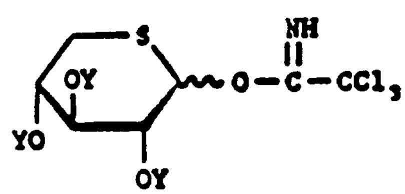

(V)

dans lesquelles Hal représente un atome d'halogène tel que Cl ou Br (l'atome de brome étant ici l'atome d'halogène préféré) et Y représente un groupe acyle aliphatique en $C_2$-$C_5$,

dans un solvant inerte, à raison de 1 mole de II pour environ 0,6 à 1,2 moles de composé III, IV ou V, en présence d'un accepteur d'acide et/ou d'un acide de Lewis et,
(ii) si nécessaire, on soumet le composé de formule I ainsi obtenu où Y est un groupe acyle aliphatique en $C_2$-$C_5$ à une réaction de désacylation à une température comprise entre 0 °C et la température de reflux du milieu réactionnel, dans un alcool inférieur en $C_1$-$C_4$ (de préférence le méthanol) en présence d'un alcoolate métallique (de préférence le méthylate de magnésium ou le méthylate de sodium), pour obtenir un composé de formule I où Y est H.

**12.** Procédé selon la revendication 11, caractérisé en outre par le fait que le composé de formule II où X représente l'atome de soufre intervenant au stade (i) est préparé selon les étapes suivantes :
a) condensation, en milieu basique fort du chlorure de diméthylaminothiocarbamoyle de formule :

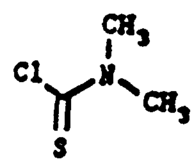

(VI)

avec un composé de formule :

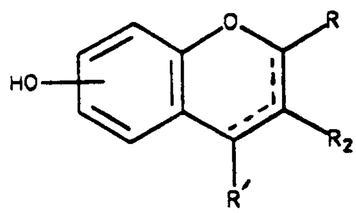

(IIa)

où R, R' et $R_2$ ont les significations indiquées ci-dessus, pour obtenir un composé de formule :

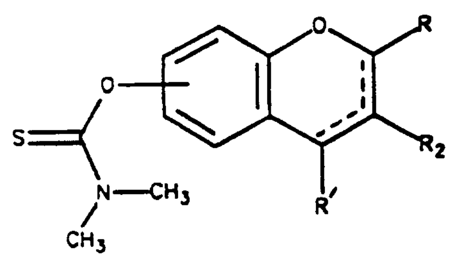

(VII)

où R, R' et $R_2$ sont définis comme ci-dessus,
b) réarrangement du composé de formule VII ainsi obtenu par chauffage, pour obtenir un composé de formule :

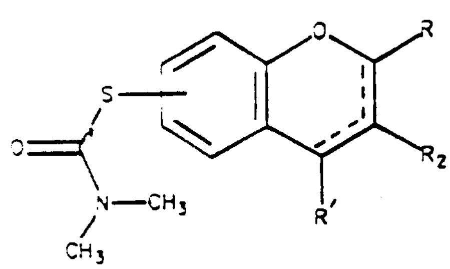

(VIII)

où R, R' et $R_2$ sont définis comme indiqués ci-dessus et,

c) traitement du composé de formule VIII ainsi obtenu par un alcoolate métallique, de préférence le méthanolate de sodium ou de magnésium, dans un alcool inférieur en $C_1$-$C_4$, le diméthylformamide ou le dioxanne, pour obtenir un composé de formule II où X = S.

**13.** Produit intermédiaire nouveau intervenant dans la synthèse des benzopyranone-β-D-thioxylosides de formule I où X représente l'atome de soufre selon l'une quelconque des revendications 1 à 3 caractérisé en ce qu'il est choisi parmi l'ensemble constitué par les composés de formule :

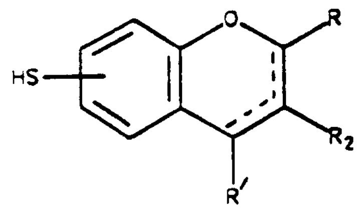

(IIb)

dans laquelle :

- l'un des substituants R ou R' représente un atome d'oxygène doublement lié au carbone cyclique et l'autre représente un groupe $R_1$,
- le symbole --- représente une double liaison conjuguée au groupe CO représenté par l'un des substituants R ou R',
- $R_1$ et $R_2$, identiques ou différents, représentent chacun un atome d'hydrogène, un groupe alkyle en $C_1$-$C_4$, un atome d'halogène, un groupe trifluorométhyle, un groupe phényle, à l'exception du groupe 3-phényle quand R représente un atome d'oxygène doublement lié au carbone cyclique, R' représente l'atome d'hydrogène, le groupe SH est lié en position 7 et le symbole --- représente une double liaison conjuguée au groupe CO représenté par le substituant R, $R_1$ et $R_2$ considérés ensemble, pouvant former avec le groupe benzopyranone auquel ils sont liés un groupe 7,8,9,10-tétrahydrodibenzo[b,d]pyran-6-one ou un groupe 1,2,3,4-tétrahydro-9H-xanthène-9-one.

**14.** Produit intermédiaire nouveau intervenant dans la synthèse des benzopyranone-β-D-thioxylosides de formule I où X représente l'atome de soufre selon l'une quelconque des revendications 1 à 3, caractérisé en ce qu'il est choisi parmi l'ensemble constitué par les composés de formule :

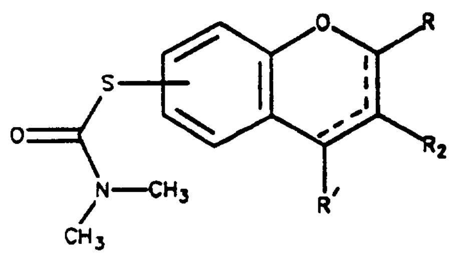

(VIII)

dans laquelle :

- $R_1$ et $R_2$, identiques ou différents, représentent chacun un atome d'hydrogène, un groupe alkyle en $C_1$-$C_4$, un atome d'halogène, un groupe trifluorométhyle, un groupe phényle, $R_1$ et $R_2$ considérés ensemble, pouvant former avec le groupe benzopyranone auquel ils sont liés un groupe 7,8,9,10-tétrahydrodibenzo[b,d]pyran-6-one ou un groupe 1,2,3,4-tétrahydro-9H-xanthène-9-one.

**Revendications pour les Etats contractants suivants : ES, GR**

**1.** Procédé pour la préparation d'un composé oside choisi parmi l'ensemble comprenant les benzopyranone-β-D-thioxylosides de formule :

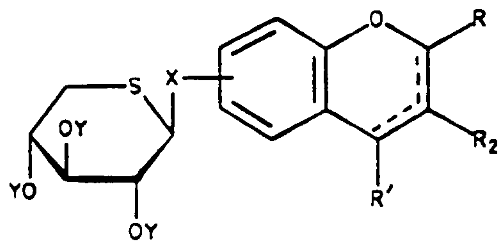

(I)

dans laquelle

- l'un des substituants R ou R' représente un atome d'oxygène doublement lié au carbone cyclique et l'autre représente un groupe $R_1$,
- le symbole --- représente une double liaison conjuguée au groupe CO représenté par l'un des substituants R ou R',
- X représente un atome de soufre ou un atome d'oxygène,
- $R_1$ et $R_2$, identiques ou différents, représentent chacun un atome d'hydrogène, un groupe alkyle en $C_1$-$C_4$, un atome d'halogène, un groupe trifluorométhyle, un groupe phényle, $R_1$ et $R_2$, considérés ensemble, pouvant former avec le groupe benzopyranone auquel ils sont liés, un groupe 7,8,9,10-tétrahydrodibenzo[b,d]pyran-6-one ou un groupe 1,2,3,4-tétrahydro-9H-xanthène-9-one; et,
- Y représente l'atome d'hydrogène ou un groupe acyle aliphatique en $C_2$-$C_5$, ledit procédé étant caractérisé en ce que :

(i) on fait réagir un composé de formule :

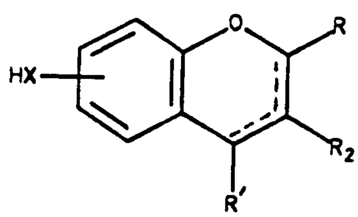

(II)

où X, R, R' et $R_2$ sont définis comme ci-dessus, avec un dérivé du thioxylose choisi parmi l'ensemble comprenant :

(i) les halogénures d'acylthioxylosyle de formule :

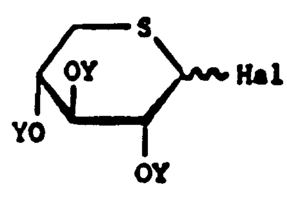

(III)

(ii) les thioxyloses peracylés de formule :

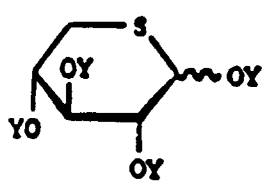

(IV)

et,
(iii) les trichloracétimidates d'acylthioxylosyle de formule :

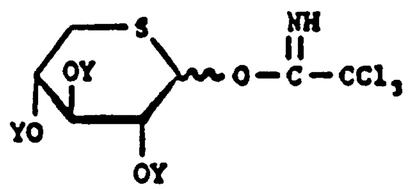

(V)

dans lesquelles Hal représente un atome d'halogène tel que Cl ou Br (l'atome de brome étant ici l'atome d'halogène préféré) et Y représente un groupe acyle aliphatique en $C_2$-$C_5$,
dans un solvant inerte, à raison de 1 mole de II pour environ 0,6 à 1,2 moles de composé III, IV ou V, en présence d'un accepteur d'acide et/ou d'un acide de Lewis et,
(ii) si nécessaire, on soumet le composé de formule I ainsi obtenu où Y est un groupe acyle aliphatique en $C_2$-$C_5$ à une réaction de désacylation à une température comprise entre 0 °C et la température de reflux du milieu réactionnel, dans un alcool inférieur en $C_1$-$C_4$ (de préférence le méthanol) en présence d'un alcoolate métallique (de préférence le méthylate de magnésium ou le méthylate de sodium), pour obtenir un composé de formule I où Y est H.

**2.** Procédé selon la revendication 1, caractérisé en outre par le fait que le composé de formule II où X représente l'atome de soufre intervenant au stade (i) est préparé selon les étapes suivantes :
a) condensation, en milieu basique fort du chlorure de diméthylaminothiocarbamoyle de formule :

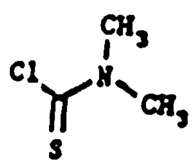

(VI)

avec un composé de formule :

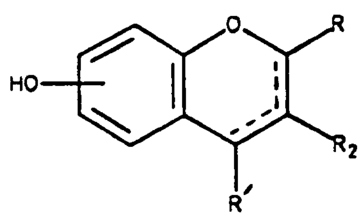

(IIa)

où R, R' et $R_2$ ont les significations indiquées ci-dessus, pour obtenir un composé de formule :

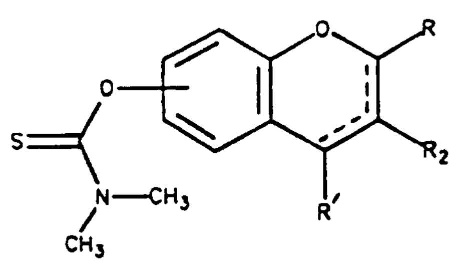

(VII)

où R, R' et $R_2$ sont définis comme ci-dessus,

b) réarrangement du composé de formule VII ainsi obtenu par chauffage, pour obtenir un composé de formule :

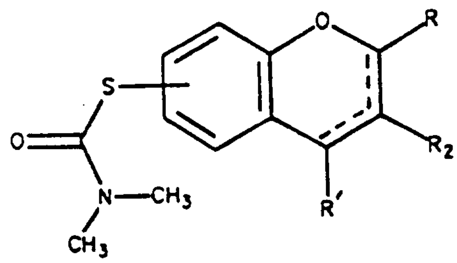

(VIII)

où R, R' et $R_2$ sont définis comme indiqué ci-dessus et,

c) traitement du composé de formule VIII ainsi obtenu par un alcoolate métallique, de préférence le méthanolate de sodium ou de magnésium, dans un alcool inférieur en $C_1$-$C_4$, le diméthylformamide ou le dioxanne, pour obtenir un composé de formule II où X = S.

**3.** Procédé selon la revendication 1 pour l'obtention d'un composé oside choisi parmi l'ensemble comprenant les benzopyran-2-one-β-D-thioxylosides de formule :

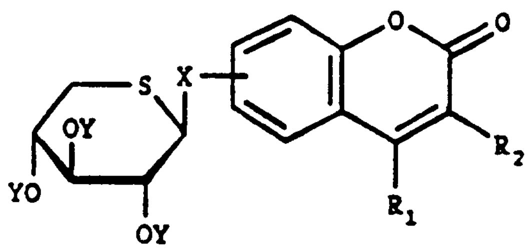

(Ia)

dans laquelle :

- X représente un atome de soufre ou un atome d'oxygène,
- $R_1$ et $R_2$, identiques ou différents, représentent chacun un atome d'hydrogène, un groupe alkyle en $C_1$-$C_4$, un atome d'halogène, un groupe trifluorométhyle, un groupe phényle, $R_1$ et $R_2$, considérés ensemble, pouvant former avec le groupe benzopyran-2-one auquel ils sont liés, un groupe 7,8,9,10-tétrahydrodibenzo[b,d]pyran-6-one; et,
- Y représente l'atome d'hydrogène ou un groupe acyle aliphatique,

ledit procédé étant caractérisé en ce que l'on fait appel à un composé de formule II selon la revendication 1 où R' est $R_1$ et R est = O.

**4.** Procédé selon la revendication 1 pour l'obtention d'un composé oside choisi parmi l'ensemble comprenant les benzopyran-4-one-β-D-thioxylosides de formule :

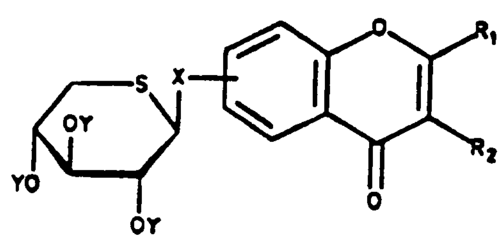

(Ib)

dans laquelle :

- X représente un atome de soufre ou un atome d'oxygène,
- $R_1$ et $R_2$, identiques ou différents, représentent chacun un atome d'hydrogène, un groupe alkyle en $C_1$-$C_4$, un atome d'halogène, un groupe trifluorométhyle, un groupe phényle, $R_1$ et $R_2$, considérés ensemble, pouvant former avec le groupe benzopyran-4-one auquel ils sont liés, un groupe 1,2,3,4-tétrahydro-9H-xanthène-9-one; et,
- Y représente l'atome d'hydrogène ou un groupe acyle aliphatique en $C_2$-$C_5$,

ledit procédé étant caractérisé en ce que l'on fait appel à un composé de formule II selon la revendication 1 où R est $R_1$ et R' est = O.

## Claims

### Claims for the following Contracting States : AT, BE, CH, DE, DK, FR, GB, IT, LI, LU, NL, SE

**1.** An oside compound selected from the group consisting of the benzopyranone-$\beta$-D-thioxylosides of the formula

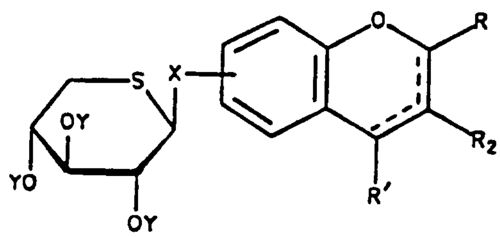

(I)

in which:
- one of the substituents R or R' is an oxygen atom double-bonded to the corresponding cyclic carbon atom and the other is a group $R_1$,
- the symbol --- represents a double bond conjugated to the CO group provided by one of the substituents R or R',
- X is a sulfur atom or an oxygen atom,
- $R_1$ and $R_2$, which are identical or different, are each a hydrogen atom, a $C_1$-$C_4$ alkyl group, a halogen atom, a trifluoromethyl group or a phenyl group, $R_1$ and $R_2$ when taken together may form a 7,8,9,10-tetrahydrodibenzo[b,d]pyran-6-one group or a 1,2,3,4-tetrahydro-9H-xanthen-9-one group with the benzopyranone group to which they are bonded, and
- Y is the hydrogen atom or a $C_2$-$C_5$ aliphatic acyl group.

**2.** An oside compound according to claim 1, selected from the group consisting of the benzopyran-2-one-$\beta$-D-thioxylosides of the formula

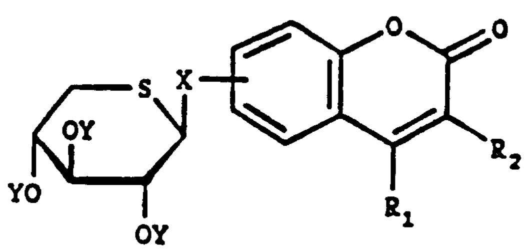

(Ia)

in which:
- X is a sulfur atom or an oxygen atom,
- $R_1$ and $R_2$, which are identical or different, are each a hydrogen atom, a $C_1$-$C_4$ alkyl group, a halogen atom, a trifluoromethyl group or a phenyl group,

  $R_1$ and $R_2$ when taken together may form a 7,8,9,10-tetrahydrodibenzo[b,d]pyran-6-one group with the benzopyran-2-one group to which they are bonded, and
- Y is the hydrogen atom or an aliphatic acyl group.

**3.** An oside compound according to claim 1, selected from the group consisting of the benzopyran-4-one-$\beta$-D-thioxylosides of the formula

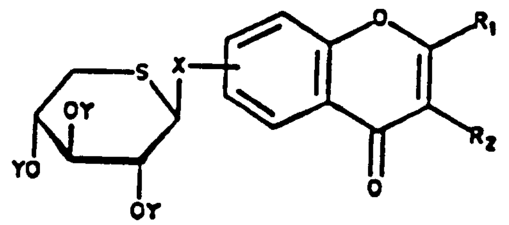

(Ib)

in which:
- X is a sulfur atom or an oxygen atom,
- $R_1$ and $R_2$, which are identical or different, are each a hydrogen atom, a $C_1$-$C_4$ alkyl group, a halogen atom, a trifluoromethyl group or a phenyl group, $R_1$ and $R_2$ when taken together may form a 1,2,3,4-tetrahydro-9H-xanthen-9-one group with the benzopyran-4-one group to which they are bonded, and
- Y is the hydrogen atom or a $C_2$-$C_5$ aliphatic acyl group.

**4.** An oside compound according to any one of claims 1 to 3, wherein X is bonded in the 7-position to the benzopyranone ring and $R_1$ and $R_2$, which are identical or different, are each a hydrogen atom, a $C_1$-$C_4$ alkyl group, a halogen atom or a phenyl group.

**5.** An oside compound according to any one of claims 1 to 4, wherein Y is the group $CH_3CO$.

**6.** 4-Methyl-2-oxo-2H-1-benzopyran-7-yl 1,5-dithio-$\beta$-D-xylopyranoside.

**7.** 3-Chloro-4-methyl-2-oxo-2H-1-benzopyran-7-yl 1,5-dithio-$\beta$-D-xylopyranoside.

**8.** 4-Ethyl-2-oxo-2H-1-benzopyran-7-yl 5-thio-$\beta$-D-xylopyranoside.

**9.** A therapeutic composition containing, in association with a physiologically acceptable excipient, at least one oside compound according to any one of claims 1 to 8.

**10.** Use of a substance according to any one of claims 1 to 8 in order to obtain an antithrombotic drug for use in therapy to combat disorders of the venous circulation.

**11.** A method of preparing a benzopyranone-$\beta$-D-thioxyloside of formula I according to claim 1, which comprises:

(i) reacting a compound of the formula

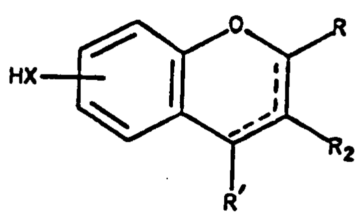

(II)

in which X, R, R' and $R_2$ are as defined above, with a thioxylose derivative selected from the group consisting of:

(i) the acylthioxylosyl halides of the formula

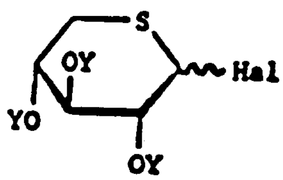

(III)

(ii) the peracylated thioxyloses of the formula

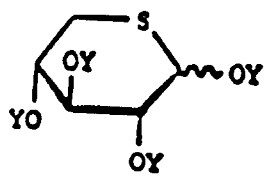

(IV)

and

(iii) the acylthioxylosyl trichloroacetimidates of the formula

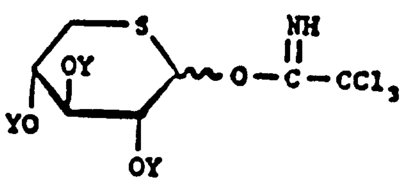

(V)

in which Hal is a halogen atom such as Cl or Br (the bromine atom being the preferred halogen atom here) and Y is a $C_2$-$C_5$ aliphatic acyl group,

in an inert solvent, at a rate of 1 mol of II to about 0.6 to 1.2 mol of compound III, IV or V, in the presence of an acid acceptor and/or a Lewis acid, and

(ii) if necessary, subjecting the resulting compound of formula I in which Y is a $C_2$-$C_5$ aliphatic acyl group to a deacylation reaction at a temperature of between 0 °C and the reflux temperature of the reaction medium, in a $C_1$-$C_4$ lower alcohol (preferably methanol), in the presence of a metal alcoholate (preferably magnesium methylate or sodium methylate), to give a compound of formula I in which Y is H.

**12.** A method according to claim 11, wherein, in addition, the compound of formula II in which X is the sulfur atom, involved in stage (i), is prepared according to the following steps:

a) condensation of dimethylaminothiocarbamoyl chloride of the formula

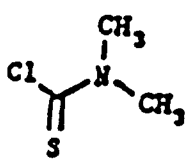

(VI)

in a strong basic medium with a compound of the formula

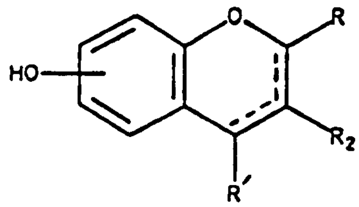

(IIa)

in which R, R' and $R_2$ are as defined above, to give a compound of the formula

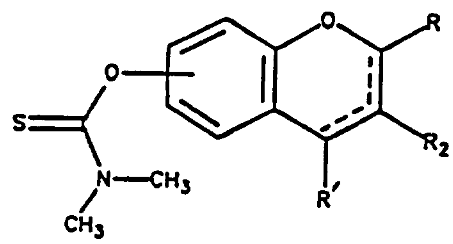

(VII)

in which R, R' and $R_2$ are as defined above,

b) rearrangement of the resulting compound of formula VII, by heating, to give a compound of the formula

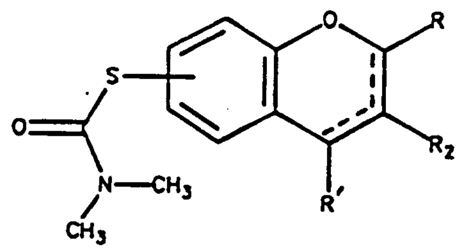

(VIII)

in which R, R' and $R_2$ are as defined above, and

c) treatment of the resulting compound of formula VIII with a metal alcoholate, preferably sodium or magnesium methylate, in a $C_1$-$C_4$ lower alcohol, dimethylformamide or dioxane, to give a compound of formula II in which X = S.

**13.** A novel intermediate involved in the synthesis of the benzopyranone-$\beta$-D-thioxylosides of formula I in which X is the sulfur atom, according to any one of claims 1 to 3, selected from the group consisting of the compounds of the formula

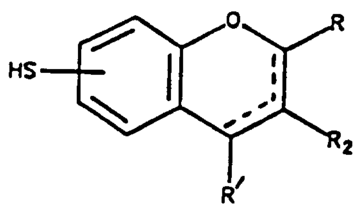

(IIb)

in which:

- one of the substituents R or R' is an oxygen atom double-bonded to the corresponding cyclic carbon atom and the other is a group $R_1$,
- the symbol --- represents a double bond conjugated to the CO group provided by one of the substituents R or R', and

- $R_1$ and $R_2$, which are identical or different, are each a hydrogen atom, a $C_1$-$C_4$ alkyl group, a halogen atom, a trifluoromethyl group or a phenyl group, with the exception of the 3-phenyl group when R is an oxygen atom double-bonded to the corresponding cyclic carbon atom, R' is the hydrogen atom, the SH group is bonded in the 7-position and the symbol --- represents a double bond conjugated to the CO group provided by the substituent R, $R_1$ and $R_2$ when taken together may form a 7,8,9,10-tetrahydrodibenzo[b,d]pyran-6-one group or a 1,2,3,4-tetrahydro-9H-xanthen-9-one group with the benzopyranone group to which they are bonded.

**14.** A novel intermediate involved in the synthesis of the benzopyranone-$\beta$-D-thioxylosides of formula I in which X is the sulfur atom, according to any one of claims 1 to 3, selected from the group consisting of the compounds of the formula

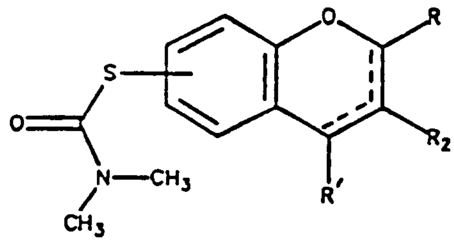

(VIII)

in which:

- $R_1$ and $R_2$, which are identical or different, are each a hydrogen atom, a $C_1$-$C_4$ alkyl group, a halogen atom, a trifluoromethyl group or a phenyl group, $R_1$ and $R_2$ when taken together may form a 7,8,9,10-tetrahydrodibenzo[b,d]pyran-6-one group or a 1,2,3,4-tetrahydro-9H-xanthen-9-one group with the benzopyranone group to which they are bonded.

**Claims for the following Contracting States : ES, GR**

**1.** A method of preparing an oside compound selected from the group consisting of the benzopyranone-$\beta$-D-thioxylosides of the formula

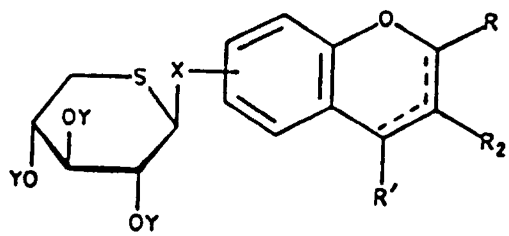

(I)

in which:

- one of the substituents R or R' is an oxygen atom double-bonded to the corresponding cyclic carbon atom and the other is a group $R_1$,
- the symbol --- represents a double bond conjugated to the CO group provided by one of the substituents R or R',
- X is a sulfur atom or an oxygen atom,
- $R_1$ and $R_2$, which are identical or different, are each a hydrogen atom, a $C_1$-$C_4$ alkyl group, a halogen atom, a trifluoromethyl group or a phenyl group, $R_1$ and $R_2$ when taken together form a 7,8,9,10-tetrahydrodibenzo[b,d]pyran-6-one group or a 1,2,3,4-tetrahydro-9H-xanthen-9-one group with the benzopyranone group to which they are bonded, and
- Y is the hydrogen atom or a $C_2$-$C_5$ aliphatic acyl group, said method comprising:

(i) reacting a compound of the formula

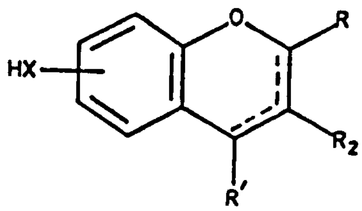

(II)

in which X, R, R' and $R_2$ are as defined above, with a thioxylose derivative selected from the group consisting of:

(i) the acylthioxylosyl halides of the formula

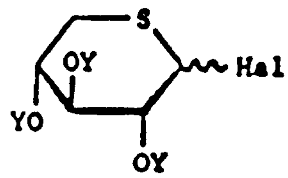

(III)

(ii) the peracylated thioxyloses of the formula

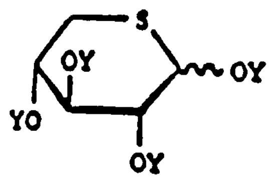

(IV)

and

(iii) the acylthioxylosyl trichloroacetimidates of the formula

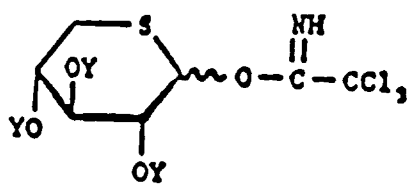

(V)

in which Hal is a halogen atom such as Cl or Br (the bromine atom being the preferred halogen atom here) and Y is a $C_2$-$C_5$ aliphatic acyl group,

in an inert solvent, at a rate of 1 mol of II to about 0.6 to 1.2 mol of compound III, IV or V, in the presence of an acid acceptor and/or a Lewis acid, and

(ii) if necessary, subjecting the resulting compound of formula I in which Y is a $C_2$-$C_5$ aliphatic acyl group to a deacylation reaction at a temperature of between 0 °C and the reflux temperature of the reaction medium, in a $C_1$-$C_4$ lower alcohol (preferably methanol), in the presence of a metal alcoholate (preferably magnesium methylate or sodium methylate), to give a compound of formula I in which Y is H.

**2.** A method according to claim 1, wherein, in addition, the compound of formula II in which X is the sulfur atom, involved in stage (i), is prepared according to the following steps:

a) condensation of dimethylaminothiocarbamoyl chloride of the formula

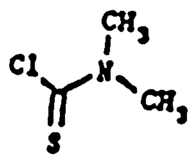

(VI)

in a strong basic medium with a compound of the formula

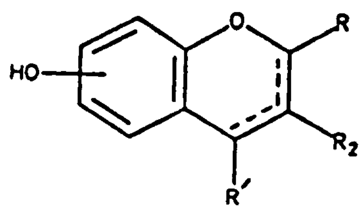

(IIa)

in which R, R' and $R_2$ are as defined above, to give a compound of the formula

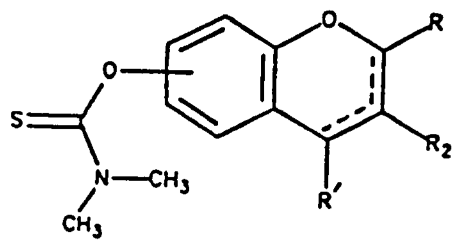

(VII)

in which R, R' and $R_2$ are as defined above,

b) rearrangement of the resulting compound of formula VII, by heating, to give a compound of the formula

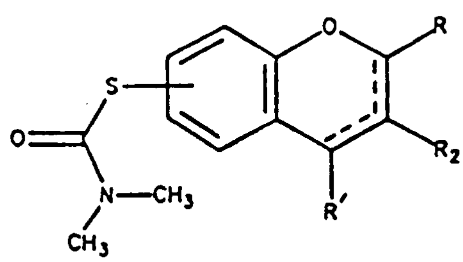

(VIII)

in which R, R' and $R_2$ are as defined above, and

c) treatment of the resulting compound of formula VIII with a metal alcoholate, preferably sodium or magnesium methylate, in a $C_1$-$C_4$ lower alcohol, dimethylformamide or dioxane, to give a compound of formula II in which X = S.

3. A method according to claim 1 for preparing an oside compound selected from the group consisting of the benzopyran-2-one-β-D-thioxylosides of the formula

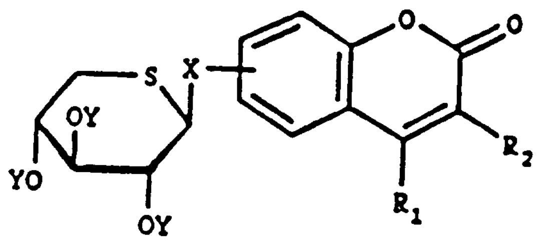

(Ia)

in which:

- X is a sulfur atom or an oxygen atom,
- $R_1$ and $R_2$, which are identical or different, are each a hydrogen atom, a $C_1$-$C_4$ alkyl group, a halogen atom, a trifluoromethyl group or a phenyl group,
  $R_1$ and $R_2$ when taken together may form a 7,8,9,10-tetrahydrodibenzo[b,d]pyran-6-one group with the benzopyran-2-one group to which they are bonded, and
- Y is the hydrogen atom or an aliphatic acyl group, said method comprising the use of a compound of formula II according to claim 1 in which R' is $R_1$ and R = O.

**4.** A method according to claim 1 for preparing an oside compound selected from the group consisting of the benzopyran-4-one-β-D-thioxylosides of the formula

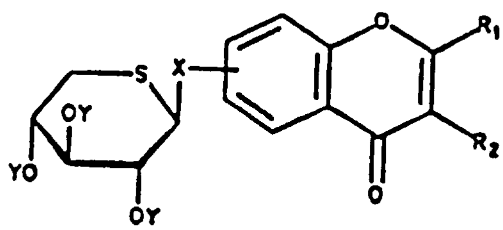

(Ib)

in which:

- X is a sulfur atom or an oxygen atom,
- $R_1$ and $R_2$, which are identical or different, are each a hydrogen atom, a $C_1$-$C_4$ alkyl group, a halogen atom, a trifluoromethyl group or a phenyl group, $R_1$ and $R_2$ when taken together may form a 1,2,3,4-tetrahydro-9H-xanthen-9-one group with the benzopyran-4-one group to which they are bonded, and
- Y is the hydrogen atom or a $C_2$-$C_5$ aliphatic acyl group, said method comprising the use of a compound of formula II according to claim 1 in which R is $R_1$ and R' = O.

**Patentansprüche**
**Patentansprüche für folgende Vertragsstaaten : AT, BE, CH, DE, DK, FR, GB, IT, LI, LU, NL, SE**

**1.** Osid-Verbindung,
**dadurch gekennzeichnet, daß**
sie aus den Benzopyranon-β-D-thioxylosiden der nachfolgenden Formel

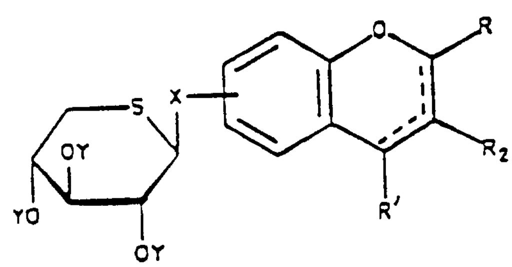

(I)

ausgewählt ist, in der

- einer der Substituenten R oder R' ein mit einer Doppelbindung an den zyklischen Kohlenstoff gebundenes Sauerstoffatom und der andere Substituent eine Gruppe $R_1$ darstellt,
- das Symbol --- eine Doppelbindung bedeutet, die mit der durch einen der Substituenten R oder R' dargestellten CO-Gruppe konjugiert ist,
- X ein Schwefelatom oder ein Sauerstoffatom darstellt,
- $R_1$ und $R_2$ gleich oder verschieden sind, und jeweils ein Wasserstoffatom, eine Alkylgruppe mit 1 bis 4 Kohlenstoffatomen, ein Halogenatom, eine Trifluormethyl-Gruppe und eine Phenyl-Gruppe darstellen, und $R_1$ und $R_2$, zusammen betrachtet, mit der Benzopyranon-Gruppe, an die sie gebunden sind, eine 7,8,9,10-Tetrahydrodibenzo[b,d]pyran-6-on- oder eine 1,2,3,4-Tetrahydro-9H-xanthen-9-on-Gruppe bilden können und
- Y ein Wasserstoffatom oder eine aliphatische Acyl-Gruppe mit 2 bis 5 Kohlenstoffatomen darstellt.

**2.** Osid-Verbindung nach Anspruch 1,
**dadurch gekennzeichnet**, **daß**
sie aus Benzopyran-2-on-$\beta$-D-thioxylosiden der nachfolgenden Formel

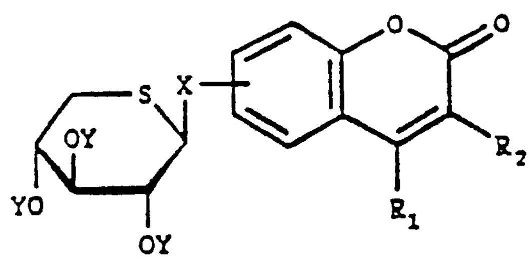

(Ia)

ausgewählt ist, in der

- X ein Schwefelatom oder ein Sauerstoffatom darstellt,
- $R_1$ und $R_2$ gleich oder verschieden sind, und jeweils ein Wasserstoffatom, eine Alkylgruppe mit 1 bis 4 Kohlenstoffatomen, ein Halogenatom, eine Trifluormethyl-Gruppe und eine Phenyl-Gruppe darstellen, und $R_1$ und $R_2$, zusammen betrachtet, mit der Benzopyran-2-on-Gruppe, an die sie gebunden sind, eine 7,8,9,10-Tetrahydrodibenzo[b,d]pyran-6-on-Gruppe bilden können und
- Y ein Wasserstoffatom oder eine aliphatische Acyl-Gruppe

darstellt.

**3.** Osid-Verbindung nach Anspruch 1,
**dadurch gekennzeichnet**, **daß**
sie aus Benzopyran-4-on-$\beta$-D-thioxylosiden der nachfolgenden Formel

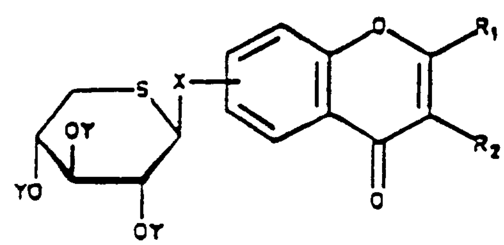

(Ib)

ausgewählt ist, in der

- X ein Schwefelatom oder ein Sauerstoffatom darstellt,
- $R_1$ und $R_2$ gleich oder verschieden sind, und jeweils ein Wasserstoffatom, eine Alkylgruppe mit 1 bis 4 Kohlenstoffatomen, ein Halogenatom, eine Trifluormethyl-Gruppe und eine Phenyl-Gruppe darstellen, und $R_1$ und $R_2$, zusammen betrachtet, mit der Benzopyran-4-on-Gruppe, an die sie gebunden sind, eine 1,2,3,4-Tetrahydro-9H-xanthen-9-on-Gruppe bilden können und

- Y ein Wasserstoffatom oder eine aliphatische Acyl-Gruppe mit 2 bis 5 Kohlenstoffatomen darstellt.

**4.** Osid-Verbindung nach einem der Ansprüche 1 bis 3,
**dadurch gekennzeichnet, daß**
X an der Position 7 am Benzopyranon-Ring gebunden ist, sowie $R_1$ und $R_2$ identisch oder verschieden sind, und jeweils ein Wasserstoffatom, eine Alkylgruppe mit 1 bis 4 Kohlenstoffatomen, ein Halogenatom oder eine Phenyl-Gruppe darstellen.

**5.** Osid-Verbindung nach einem der Ansprüche 1 bis 4,
**dadurch gekennzeichnet, daß**
Y die Gruppe $CH_3CO$ darstellt.

**6.** 4-Methyl-2-oxo-2H-1-benzopyran-7-yl-1,5-dithio-β-D-xylopyranosid.

**7.** 3-Chlor-4-methyl-2-oxo-2H-1-benzopyran-7-yl-1,5-dithio-β-D-xylopyranosid.

**8.** 4-Ethyl-2-oxo-2H-1-benzopyran-7-yl-5-thio-β-D-xylopyranosid.

**9.** Therapeutische Zusammensetzung,
**dadurch gekennzeichnet, daß**
sie in Verbindung mit einem physiologisch annehmbaren Excipienten mindestens eine Osid-Verbindung nach einem der Ansprüche 1 bis 8 enthält.

**10.** Verwendung einer Substanz nach einem der Ansprüche 1 bis 8 zum Erhalt eines antithrombotischen Medikaments, zur therapeutischen Verwendung bei Störungen des Venenkreislaufs.

**11.** Verfahren zur Herstellung eines Benzopyranon-β-D-thioxylosids der Formel (I) nach Anspruch 1,
**dadurch gekennzeichnet, daß**
es
(i) die Umsetzung einer Verbindung der Formel

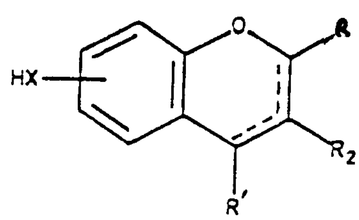

(II)

,

in der X, R, R' und $R_2$ die vorstehend angegebene Bedeutung haben, mit einem aus den nachfolgenden Thioxylosederivaten ausgewählten Verbindung

(i) Acylthioxylosylhalogeniden der Formel

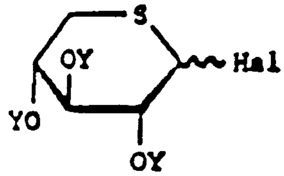

(III)

,

(ii) Peracylthioxylosen der Formel

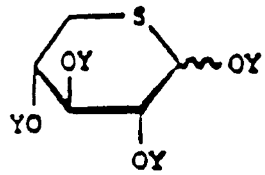

(IV)

und

(iii) Trichloracetimidatacylthioxylosylen der Formel

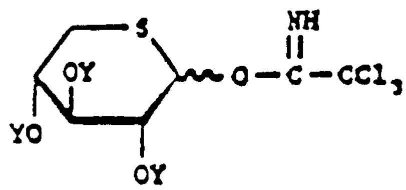

(V)

,

in denen Hal ein Halogenatom wie Cl oder Br (das Bromatom ist von den Halogenatomen bevorzugt) und Y eine aliphatische Acylgruppe mit 2 bis 5 Kohlenstoffatomen darstellt,

in einem inerten Lösungsmittel, in einem Verhältnis von 1 Mol Verbindung (II) pro etwa 0,6 bis 1,2 Mol der Verbindungen (III), (IV) oder (V), in Anwesenheit eines Säureakzeptors und/oder einer Lewis-Säure umfaßt und,

(ii) falls erforderlich, die auf diese Weise erhaltene Verbindung der Formel (I), in der Y eine aliphatische Acylgruppe mit 2 bis 5 Kohlenstoffatomen ist, einer Deacylierungsreaktion bei einer Temperatur zwischen 0 °C und der Rückflußtemperatur der Reaktionsmischung, in einem niederen Alkohol mit 1 bis 4 Kohlenstoffatomen (vorzugsweise Methanol) in Anwesenheit eines Metallalkoholats (vorzugsweise Magnesiummethylat oder Natriummethylat) unterzieht, wobei eine Verbindung der Formel (I) erhalten wird, in der Y Wasserstoff ist.

**12.** Verfahren nach Anspruch 11,

**dadurch gekennzeichnet**, **daß**

die in der Stufe (i) eingesetzte Verbindung der Formel (II), in der X ein Schwefelatom darstellt, gemäß folgenden Schritten hergestellt wird:

a) Kondensation von Dimethylaminothiocarbamoylchlorid der Formel

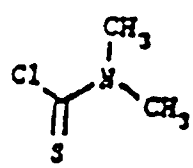

(VI)

in stark basischem Milieu mit einer Verbindung der Formel

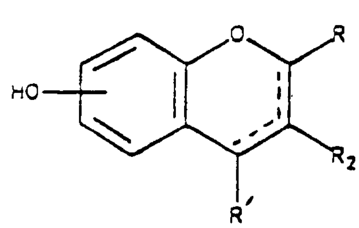

(IIa)

,

in der R, R' und $R_2$ die vorstehend angegebene Bedeutung haben, wobei eine Verbindung der Formel

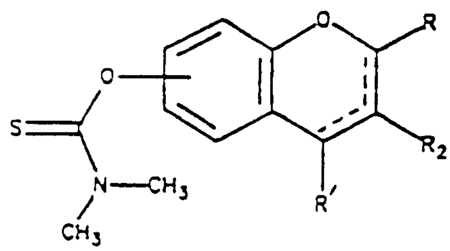

(VII)

erhalten wird, in der R, R' und $R_2$ die vorstehend angegebene Bedeutung haben,

b) Umlagerung der Verbindung der Formel (VII) durch Erhitzen, wobei eine Verbindung der Formel

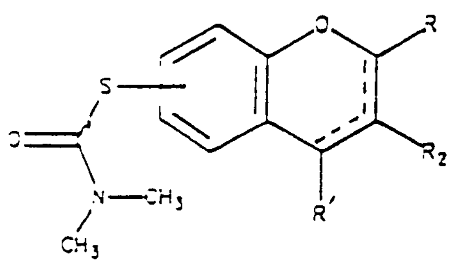

(VIII)

erhalten wird, in der R, R' und $R_2$ die vorstehend angegebene Bedeutung haben, und

c) Behandlung der auf diese Weise erhaltenen Verbindung der Formel (VIII) mit einem Metallalkoholat, vorzugsweise Natriummethanolat oder Magnesiummethanolat, in einem niederen Alkohol mit 1 bis 4 Kohlenstoffatomen in Dimethylformamid oder Dioxan, wobei eine Verbindung der Formel (II), in der X Schwefel ist, erhalten wird.

**13.** Neues zur Synthese von Benzopyranon-$\beta$-D-thioxylosiden der Formel (I) geeignetes Zwischenprodukt, in dem X ein Schwefelatom ist, nach einem der Ansprüche 1 bis 3,
**dadurch gekennzeichnet, daß**
es aus Verbindungen der nachfolgenden Formel

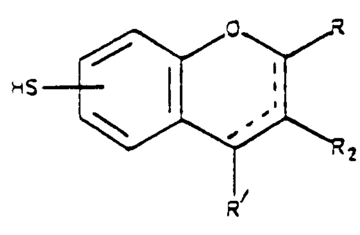

(IIb)

ausgewählt ist, in der

- einer der Substituenten R oder R' ein mit einer Doppelbindung an den zyklischen Kohlenstoff gebundenes Sauerstoffatom und der andere Substituent eine Gruppe $R_1$ darstellt,
- das Symbol --- eine Doppelbindung bedeutet, die mit der durch einen der Substituenten R oder R' dargestellten CO-Gruppe konjugiert ist,
- $R_1$ und $R_2$ gleich oder verschieden sind, und jeweils ein Wasserstoffatom, eine Alkylgruppe mit 1 bis 4 Kohlenstoffatomen, ein Halogenatom, eine Trifluormethyl-Gruppe und eine Phenyl-Gruppe mit der Ausnahme der 3-Phenyl-Gruppe, wenn R ein an den zyklischen Kohlenstoff mit einer Doppelbindung gebundenes Sauerstoffatom ist, R' ein Wasserstoffatom ist, die SH-Gruppe in Position 7 ist und das Symbol --- eine Doppelbindung bedeutet, die mit der durch einen der Substituenten R, $R_1$ und $R_2$ dargestellten CO-Gruppe konjugiert ist, die zusammen betrachtet, mit

der Benzopyranon-Gruppe, an die sie gebunden sind, eine 7,8,9,10-Tetrahydrodibenzo[b,d]pyran-6-on- oder eine 1,2,3,4-Tetrahydro-9H-xanthen-9-on-Gruppe bilden können.

**14.** Neues zur Synthese von Benzopyranon-$\beta$-D-thioxylosiden der Formel (I), in der X ein Schwefelatom darstellt, geeignete Zwischenprodukte nach einem der Ansprüche 1 bis 3,
**dadurch gekennzeichnet**, **daß**
es aus Verbindungen der Formel

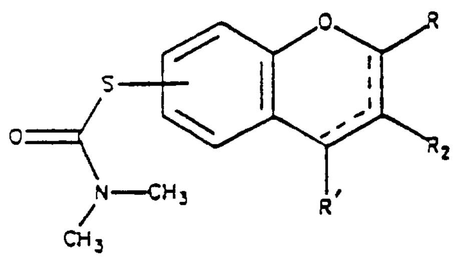

(VIII)

ausgewählt ist, in der

- $R_1$ und $R_2$ gleich oder verschieden sind, und jeweils ein Wasserstoffatom, eine Alkylgruppe mit 1 bis 4 Kohlenstoffatomen, ein Halogenatom, eine Trifluormethyl-Gruppe und eine Phenyl-Gruppe darstellen, und $R_1$ und $R_2$, zusammen betrachtet, mit der Benzopyranon-Gruppe, an die sie gebunden sind, eine 7,8,9,10-Tetrahydrodibenzo[b,d]pyran-6-on- oder eine 1,2,3,4-Tetrahydro-9H-xanthen-9-on-Gruppe bilden können.

**Patentansprüche für folgende Vertragsstaaten : ES, GR**

**1.** Verfahren zur Herstellung einer aus den Benzopyranon-$\beta$-D-thioxylosiden der nachfolgenden Formel

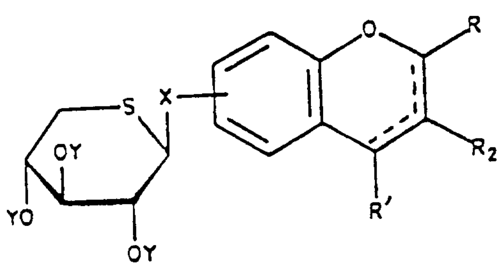

(I)

ausgewählten Osid-Verbindung, in der

- einer der Substituenten R oder R' ein mit einer Doppelbindung an den zyklischen Kohlenstoff gebundenes Sauerstoffatom und der andere Substituent eine Gruppe $R_1$ darstellt,
- das Symbol --- eine Doppelbindung bedeutet, die mit der durch einen der Substituenten R oder R' dargestellten CO-Gruppe konjugiert ist,
- X ein Schwefelatom oder ein Sauerstoffatom darstellt,
- $R_1$ und $R_2$ gleich oder verschieden sind, und jeweils ein Wasserstoffatom, eine Alkylgruppe mit 1 bis 4 Kohlenstoffatomen, ein Halogenatom, eine Trifluormethyl-Gruppe und eine Phenyl-Gruppe darstellen, und $R_1$ und $R_2$, zusammen betrachtet, mit der Benzopyranon-Gruppe, an die sie gebunden sind, eine 7,8,9,10-Tetrahydrodibenzo[b,d]pyran-6-on- oder eine 1,2,3,4-Tetrahydro-9H-Xanthen-9-on-Gruppe bilden können und
- Y ein Wasserstoffatom oder eine aliphatische Acyl-Gruppe mit 2 bis 5 Kohlenstoffatomen darstellt,

**dadurch gekennzeichnet**, **daß**
es

(i) die Umsetzung einer Verbindung der Formel

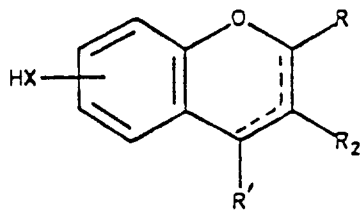

(II)

,

in der X, R, R' und $R_2$ die vorstehend angegebene Bedeutung haben, mit einem aus den nachfolgenden Thioxylosederivaten ausgewählten Verbindung

(i) Acylthioxylosylhalogeniden der Formel

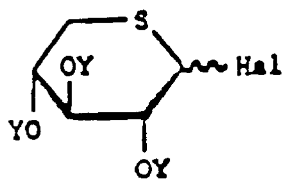

(III)

,

(ii) Peracylthioxylosen der Formel

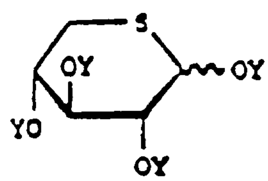

(IV)

und

(iii) Trichloracetimidatacylthioxylosylen der Formel

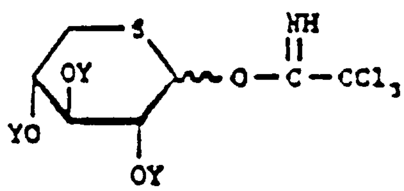

(V)

,

in denen Hal ein Halogenatom wie Cl oder Br (das Bromatom ist von den Halogenatomen bevorzugt) und Y eine aliphatische Acylgruppe mit 2 bis 5 Kohlenstoffatomen darstellt,

in einem inerten Lösungsmittel, in einem Verhältnis von 1 Mol Verbindung (II) pro etwa 0,6 bis 1,2 Mol der Verbindungen (III), (IV) oder (V), in Anwesenheit eines Säureakzeptors und/oder einer Lewis-Säure umfaßt und,

(ii) falls erforderlich, die auf diese Weise erhaltene Verbindung der Formel (I), in der Y eine aliphatische Acylgruppe mit 2 bis 5 Kohlenstoffatomen ist, einer Deacylierungsreaktion bei einer Temperatur zwischen 0 °C und der Rückflußtemperatur der Reaktionsmischung, in einem niederen Alkohol mit 1 bis 4 Kohlenstoffatomen (vorzugsweise Methanol) in Anwesenheit eines Metallalkoholats (vorzugsweise Magnesiummethylat oder Natriummethylat) unterzieht, wobei eine Verbindung der Formel (I) erhalten wird, in der Y Wasserstoff ist.

**2.** Verfahren nach Anspruch 1,
**dadurch gekennzeichnet**, **daß**
die in der Stufe (i) eingesetzte Verbindung der Formel (II), in der X ein Schwefelatom darstellt, gemäß folgenden Schritten hergestellt wird:

a) Kondensation von Dimethylaminothiocarbamoylchlorid der Formel

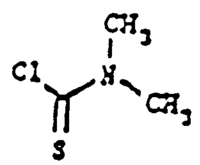

(VI)

in stark basischem Milieu mit einer Verbindung der Formel

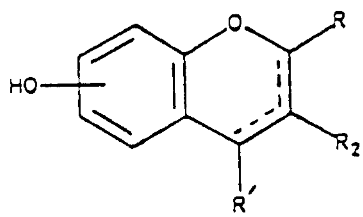

(IIa)

,

in der R, R' und $R_2$ die vorstehend angegebene Bedeutung haben, wobei eine Verbindung der Formel

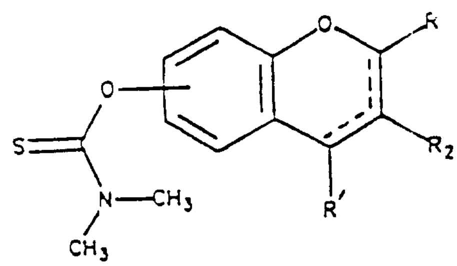

(VII)

erhalten wird, in der R, R' und $R_2$ die vorstehend angegebene Bedeutung haben,

b) Umlagerung der Verbindung der Formel (VII) durch Erhitzen, wobei eine Verbindung der Formel

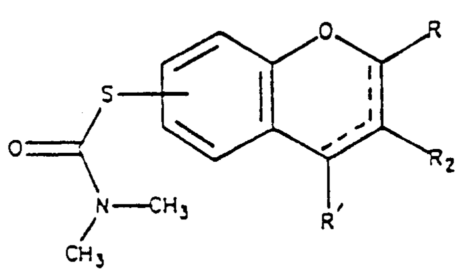

(VIII)

erhalten wird, in der R, R' und $R_2$ die vorstehend angegebene Bedeutung haben, und

c) Behandlung der auf diese Weise erhaltenen Verbindung der Formel (VIII) mit einem Metallalkoholat, vorzugsweise Natriummethanolat oder Magnesiummethanolat, in einem niederen Alkohol mit 1 bis 4 Kohlenstoffatomen in Dimethylformamid oder Dioxan, wobei eine Verbindung der Formel (II), in der X Schwefel ist, erhalten wird.

**3.** Verfahren nach Anspruch 1 zur Herstellung einer aus Benzopyran-2-on-β-D-thioxylosiden der Formel

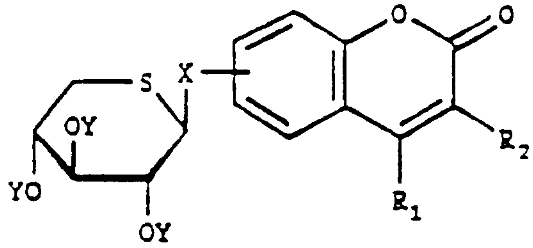

(Ia)

,

ausgewählten Osid-Verbindung, in der

- X ein Schwefelatom oder ein Sauerstoffatom darstellt,
- $R_1$ und $R_2$ gleich oder verschieden sind, und jeweils ein Wasserstoffatom, eine Alkylgruppe mit 1 bis 4 Kohlenstoffatomen, ein Halogenatom, eine Trifluormethyl-Gruppe und eine Phenyl-Gruppe darstellen, und $R_1$ und $R_2$, zusammen betrachtet, mit der Benzopyran-2-on-Gruppe, an die sie gebunden sind, eine 7,8,9,10-Tetrahydrodibenzo[b,d]pyran-6-on-Gruppe bilden können, und
- Y ein Wasserstoffatom oder eine aliphatische Acyl-Gruppe darstellt,

**dadurch gekennzeichnet**, **daß**
eine Verbindung der Formel (II) nach Anspruch 1 eingesetzt wird, in der R' = $R_1$ und R Sauerstoff ist.

**4.** Verfahren nach Anspruch 1 zur Herstellung einer aus Benzopyran-4-on-β-D-thioxylosiden der Formel

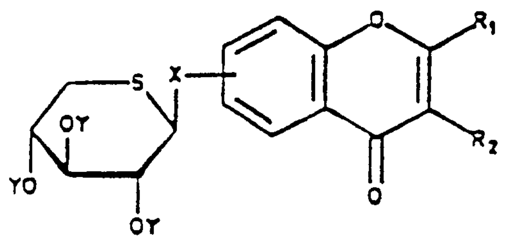

(Ib)

ausgewählten Osid-Verbindung, in der

- X ein Schwefelatom oder ein Sauerstoffatom darstellt,
- $R_1$ und $R_2$ gleich oder verschieden sind, und jeweils ein Wasserstoffatom, eine Alkylgruppe mit 1 bis 4 Kohlenstoffatomen, ein Halogenatom, eine Trifluormethyl-Gruppe und eine Phenyl-Gruppe darstellen, und $R_1$ und $R_2$, zusammen betrachtet, mit der Benzopyran-2-on-Gruppe, an die sie gebunden sind, eine 1,2,3,4-Tetrahydro-9H-xanthen-9-on-Gruppe bilden können, und
- Y ein Wasserstoffatom oder eine aliphatische Acyl-Gruppe mit 2 bis 5 Kohlenstoffatomen darstellt,

**dadurch gekennzeichnet**, **daß**
eine Verbindung der Formel (II) nach Anspruch 1 eingesetzt wird, in der R = $R_1$ und R' Sauerstoff ist.